# EUROPEAN PATENT APPLICATION

(11) **EP 2 596 706 A1**
(43) Date of publication of application: **29.05.2013**
(21) Application number: 11820056.7
(22) Date of filing: 26.08.2011
(51) Int. Cl.: A23L 3/37, C07K 14/46

(54) **METHOD FOR INCREASING THERMAL HYSTERESIS ACTIVITY, METHOD FOR REDUCING THERMAL INACTIVATION OF THERMAL HYSTERESIS ACTIVITY, AND COMPOSITION FOR INCREASING THERMAL HYSTERESIS ACTIVITY**

(30) Priority: 26.08.2010 JP 2010189723
(71) Applicant: Nichirei Foods Inc., Tokyo 104-8402 (JP)
(72) Inventor: OYAIZU Shinichi, Tokyo 104-8402 (JP); INOUE Toshifumi, Chiba-shi Chiba 261-0002 (JP); KAJI Daisuke, Chiba-shi Chiba 261-0002 (JP); OOMORI Toshinobu, Chiba-shi Chiba 261-0002 (JP); KOIZUMI Takeshi, Chiba-shi Chiba 261-0002 (JP); ISHII Hirotaka, Chiba-shi Chiba 261-0002 (JP)
(74) Representative: Cabinet Plasseraud
(86) International application number: PCT/JP2011/069338
(87) International publication number: WO 2012/026596

(57) **Abstract**

The present invention relates to a method of increasing the thermal hysteresis activity exhibited by a composition containing an antifreeze protein and water, wherein the method includes incorporating the antifreeze protein and any one or more substances selected from the following additive group A into the water. According to the present invention, the method of further increasing the thermal hysteresis activity inherently possessed by an antifreeze protein can be provided.

Additive group A: an organic acid, an organic acid salt, an inorganic salt, an amino acid, an amino acid salt, an ammonium salt, a sugar, a sugar alcohol, urea, guanidine hydrochloride, spermidine, spermine, and N,N-dimethylformamide.

## Description

### TECHNICAL FIELD

The present invention relates to a method of increasing thermal hysteresis activity, a method of reducing thermal inactivation of the thermal hysteresis activity, and a composition for increasing the thermal hysteresis activity.
Priority is claimed on Japanese Patent Application No. 2010-189723, filed August 26, 2010, the content of which is incorporated herein by reference.

### BACKGROUND ART

Antifreeze proteins (AFP) have an action of binding to the crystal surfaces of ice in an aqueous solution and thereby suppressing the growth of the crystals of ice, and an action of lowering the freezing point of the aqueous solution. Antifreeze proteins having such properties originate from those discovered from the blood plasma of a fish that inhabits the Antarctic in 1969, and to the present, various antifreeze proteins have been discovered from a wide range of domains of the living world such as fishes, insects, plants, microorganisms, and Basidiomycetes.

Aqueous solutions containing the antifreeze proteins are also known to have a unique property called thermal hysteresis (a property in which the melting point and the freezing point are different).
First, an aqueous solution containing an antifreeze protein is cooled, and the aqueous solution is completely frozen to ice (Step 1). Next, when the temperature is slowly elevated, the melting point is determined as a temperature at which the ice melts (Step 2). Subsequently, when the temperature is slowly lowered again, a temperature zone is observed in which the crystals of ice do not grow despite the temperature being lower than or equal to the melting point, and if the temperature is further lowered, the freezing point is determined as a temperature at which the crystals of ice grow instantaneously (Step 3). The temperature difference between the melting point and the freezing point at this time is used as an indicator exhibiting the activity of the antifreeze protein, and the temperature difference is referred to as thermal hysteresis activity (ΔT).

The relationship between the concentration of the antifreeze protein in the aqueous solution and the thermal hysteresis activity of the solution exhibits a positive correlation with an increase in the thermal hysteresis activity to a certain concentration; however, if the concentration exceeds that certain level, the thermal hysteresis activity increases no further even if the concentration is further increased.

It is known that the thermal hysteresis activity exhibited by known fish-derived antifreeze proteins is about 0.1°C to 2°C, but the thermal hysteresis activity exhibited by insect-derived antifreeze proteins is up to about 10°C. There is no known instance of obtaining a thermal hysteresis activity equivalent to the thermal hysteresis activity exhibited by an insect-derived antifreeze protein (for example, ΔT = 5°C), even if the concentration of a fish-derived antifreeze protein contained in an aqueous solution is increased.

An investigation is underway regarding incorporating such an antifreeze protein having a thermal hysteresis activity into ice cream or frozen foods, and thereby smoothening the melt-in-the-mouth of the ice cream or preventing the deterioration of food materials caused by ice crystal growth in frozen foods. In order to use antifreeze proteins for industrial applications, a method by which large amounts of antifreeze proteins can be supplied at low cost is needed, and for example, a method of producing a fish-derived antifreeze protein in an amount in the order of grams has been disclosed (see Patent Document 1).

### Prior Art Documents

### Patent Documents

[Patent Document 1] Japanese Laid-Open Patent Application No. 2004-083546

### DISCLOSURE OF INVENTION

### PROBLEMS TO BE SOLVED BY THE INVENTION

However, since the thermal hysteresis activity exhibited by antifreeze proteins other than the insect-derived antifreeze proteins is not necessarily sufficient, a method of further increasing the thermal hysteresis activity is desired. Furthermore, in the case of assuming sterilization of food products containing antifreeze proteins by heating, it is desired that the decrease in the thermal hysteresis activity caused by heating as described above can be suppressed.

The present invention was achieved in view of such circumstances, and it is an object of the invention to provide a method of further increasing the thermal hysteresis activity exhibited by an antifreeze protein.
In addition, the present invention was achieved in view of such circumstances, and in the case of assuming sterilization of food products containing antifreeze proteins by heating, it is another object of the invention to provide a method of suppressing a decrease of the thermal hysteresis activity caused by the heating.
Furthermore, the present invention was achieved in view of the circumstances described above, and it is still another object of the invention to provide a composition for increasing the thermal hysteresis activity, which is capable of enhancing the thermal hysteresis activity exhibited by an antifreeze protein, or capable of suppressing a decrease caused by heating in the thermal hysteresis activity exhibited by an antifreeze protein.

### MEANS TO SOLVE THE PROBLEMS

The method of increasing the thermal hysteresis activity described in Claim 1 of the invention is a method of increasing the thermal hysteresis activity exhibited by a composition containing an antifreeze protein and water, in which the antifreeze protein and any one or more selected from the following additive group A are dissolved in the water:
Additive group A: an organic acid, an organic acid salt, an inorganic salt, an amino acid, an amino acid salt, an ammonium salt, a sugar, a sugar alcohol, urea, guanidine hydrochloride, spermidine, spermine, and N,N-dimethylformamide.

The method of reducing thermal deactivation of the thermal hysteresis activity described in Claim 2 of the invention is a method of decreasing the extent to which the thermal hysteresis activity exhibited by a composition containing an antifreeze protein and water decreases after the heating treatment, in which the antifreeze protein and any one or more selected from the following additive group B are dissolved in the water:
Additive group B: an organic acid, an organic acid salt, an inorganic salt, an amino acid, an amino acid salt, an ammonium salt, a sugar, a sugar alcohol, urea, guanidine hydrochloride, acetone, dioxane, 2-chloroethanol, and N,N-dimethylformamide.

The composition for increasing the thermal hysteresis activity described in Claim 3 of the invention is a composition for increasing the thermal hysteresis activity containing an antifreeze protein and an additive, and is a composition for increasing the thermal hysteresis activity, in which the additive includes any one or more of an organic acid, an organic acid salt, an inorganic salt, an amino acid, an amino acid salt, an ammonium salt, a sugar, a sugar alcohol, urea, guanidine hydrochloride, spermidine, spermine, acetone, dioxane, 2-chloroethanol and N,N-dimethylformamide.

The method of increasing the thermal hysteresis activity described in Claim 4 of the invention is such that the antifreeze protein mentioned in Claim 1 is a fish-derived antifreeze protein.
The method of reducing thermal deactivation of the thermal hysteresis activity described in Claim 5 of the invention is such that the antifreeze protein mentioned in Claim 2 is a fish-derived antifreeze protein.
The composition for increasing the thermal hysteresis activity described in Claim 6 of the invention is such that the antifreeze protein mentioned in Claim 3 is a fish-derived antifreeze protein.

That is, the invention includes the following embodiments.
(1) A method of increasing the thermal hysteresis activity exhibited by a composition containing an antifreeze protein and water, the method including incorporating the antifreeze protein and any one or more substances selected from the following additive group A into the water:
   additive group A: an organic acid, an organic acid salt, an inorganic salt, an amino acid, an amino acid salt, an ammonium salt, a sugar, a sugar alcohol, urea, guanidine hydrochloride, spermidine, spermine, and N,N-dimethylformamide.
(2) A method of reducing the post-heat treatment deactivation of the thermal hysteresis activity exhibited by a composition containing an antifreeze protein and water, the method including incorporating the antifreeze protein and any one or more substances selected from the following additive group B into the water:
   additive group B: an organic acid, an organic acid salt, an inorganic salt, an amino acid, an amino acid salt, an ammonium salt, a sugar, a sugar alcohol, urea, guanidine hydrochloride, acetone, dioxane, 2-chloroethanol, and N,N-dimethylformamide.
(3) A composition for increasing thermal hysteresis activity, the composition containing an antifreeze protein, water and an additive, the additive including any one or more substances selected from the group consisting of an organic acid, an organic acid salt, an inorganic salt, an amino acid, an amino acid salt, an ammonium salt, a sugar, a sugar alcohol, urea, guanidine hydrochloride, spermidine, spermine, acetone, dioxane, 2-chloroethanol, and N,N-dimethylformamide.
(4) The method of increasing the thermal hysteresis activity as described in (1), wherein the antifreeze protein is a fish-derived protein.
(5) The method of reducing the post-heat treatment deactivation of the thermal hysteresis activity as described in (2), wherein the antifreeze protein is a fish-derived protein.
(6) The composition for increasing thermal hysteresis activity as described in (3), wherein the antifreeze protein is a fish-derived protein.

(7) The method of increasing the thermal hysteresis activity as described in (4), wherein the antifreeze protein is a Type I antifreeze protein.
(8) The method of increasing the thermal hysteresis activity as described in (4), wherein the antifreeze protein is a Type II antifreeze protein.
(9) The method of increasing the thermal hysteresis activity as described in (4), wherein the antifreeze protein is a Type III antifreeze protein.
(10) The method of increasing the thermal hysteresis activity as described in (4), wherein the antifreeze protein is an AFGP type antifreeze protein.

(11) The method of reducing the post-heat treatment deactivation of the thermal hysteresis activity as described in (5), wherein the antifreeze protein is a Type I antifreeze protein.
(12) The method of reducing the post-heat treatment deactivation of the thermal hysteresis activity as described in (5), wherein the antifreeze protein is a Type II antifreeze protein.
(13) The method of reducing the post-heat treatment deactivation of the thermal hysteresis activity as described in (5), wherein the antifreeze protein is a Type III antifreeze protein.
(14) The method of reducing the post-heat treatment deactivation of the thermal hysteresis activity as described in (5), wherein the antifreeze protein is an AFGP type antifreeze protein.

(15) The method of increasing the thermal hysteresis activity as described in (1), wherein the concentration of the antifreeze protein contained in the mixture of water, the antifreeze protein and the additive is 0.01 mass% to 10 mass%.
(16) The method of reducing the post-heat treatment deactivation of the thermal hysteresis activity as described in (2), wherein the concentration of the antifreeze protein contained in the mixture of water, the antifreeze protein and the additive is 0.01 mass% to 10 mass%.
(17) The composition for increasing thermal hysteresis activity as described in (3), wherein the concentration of the antifreeze protein contained in the composition for increasing thermal hysteresis activity is 0.01 mass% to 10 mass%.

### Advantageous Effects of Invention

According to the method of increasing thermal hysteresis activity of the invention, the thermal hysteresis activity exhibited by an antifreeze protein can be further increased.
Furthermore, according to the method of reducing the thermal deactivation of thermal hysteresis activity of the invention, when it is assumed that food containing an antifreeze protein is sterilized by heating, the decrease in the thermal hysteresis activity exhibited by the antifreeze protein caused by the heating can be suppressed.
Moreover, according to the composition for increasing thermal hysteresis activity of the invention, the thermal hysteresis activity exhibited by an antifreeze protein can be further increased, or the decrease of the thermal hysteresis activity exhibited by an antifreeze protein caused by heating can be suppressed.

### BRIEF DESCRIPTION OF THE DRAWINGS

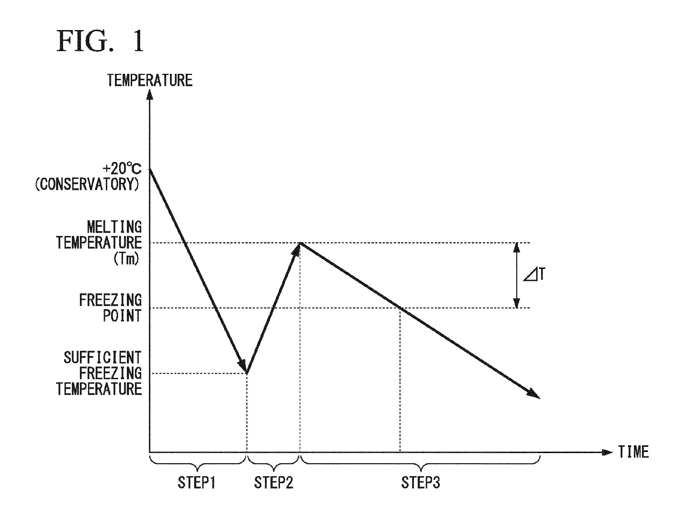

FIG. 1 is a diagram illustrating the change in temperature of a sample upon the measurement of thermal hysteresis activity.

### EMBODIMENTS OF THE INVENTION

Hereinafter, the present invention will be described in detail.

### <Method of increasing thermal hysteresis activity>

The method of increasing thermal hysteresis activity according to a first embodiment of the invention is a method of increasing the thermal hysteresis activity exhibited by a composition containing an antifreeze protein and water, and is a method of incorporating the antifreeze protein and any one or more substances selected from the following additive group A into the water:
additive group A: an organic acid, an organic acid salt, an inorganic salt, an amino acid, an amino acid salt, an ammonium salt, a sugar, a sugar alcohol, urea, guanidine hydrochloride, spermidine, spermine, and N,N-dimethylformamide.

The water may be an aqueous solution or a water-containing material, which both contain other substances, as long as the effects of the invention are not impaired. The mixture of the invention is a mixture obtained by mixing water (including the aqueous solution and the water-containing material as well), the antifreeze protein, and the additive.

The mixture preferably contains water as a main component. At this time, since the mixture includes the additive, the thermal hysteresis activity of the mixture can be further increased only by incorporating the antifreeze protein, as compared to the case of not incorporating the additive. That is, the additive offers an effect of enhancing (promoting) the thermal hysteresis activity inherently possessed by an antifreeze protein.

The antifreeze protein for the method of increasing thermal hysteresis activity of the invention is not particularly limited as long as the antifreeze protein has the thermal hysteresis activity, and any known antifreeze protein can be used.

Examples of the antifreeze protein include antifreeze proteins derived from fishes that belong to the genera Myoxocephalus, Gymnocanthus, Hemilepidotus, Furcina, Hypomesus, Spirinchus, Mallotus, Pleurogrammus, Sebastes, Clupea, Limanda, Liopsetta, Clidoderma, Cleisthenes, Microstomus, Lepidopsetta, Platichthys, Kareius, Eopsetta, Gadus, Theragra, Ammodytes, Hypoptychus, Trachurus, Brachyopsis, Pholis, Opisthocentrus, Zoarces, Ascoldia, and Pholidapus.

Specifically, examples of the antifreeze protein include antifreeze proteins which are extractable from protein extraction sources derived from fishes that belong to Myoxocephalus stelleri Tilesius, Gymnocanthus herzensteini Jordan et Starks, Myoxocephalus polyacanthocephalus (Pallas), Hemilepidotus jordani Bean, Furcina osimae Jordan et Starks, Hypomesus pretiosus japonicas (Brevoort), Hypomesus olidus (Dallas), Spirinchus lanceolatus (Hikita), Mallotus villosus (Muller), Pleurogrammus monopterygius (Pallas), Sebastes steindachneri Hilgendorf, Clupea pallasii Valenciennes, Limanda herzensteini Jordan et Snyder, Limanda punctatissima (Steindachner), Limanda schrenki Schmidt, Limanda aspera (Pallas), Liopsetta obscura (Herzenstein), Clidoderma asperrimum (Temminck et Schlegel), Cleisthenes pinetorumherzensteini (Schmidt), Microctomus achne (Jordan et Starks), Lepidopsetta mochigarei Snyder, Platichthys stellatus (Pallas), Kareius bicoloratus (Basilewsky), Eopsetta grigorjewi (Herzenstein), Gadus macrocephalus Tilesius, Theragra chalcogramma (Pallas), Ammodytes personatus Girard, Hypoptychus dybowskii Steindachner, Trachurus japonicas (Temminck et Schlegel), Brachyopsis, rostratus (Tilesius), Pholis picta (Kner), Opisthocentrus ocellatus (Tilesius), Zoarces elongatus Kner, Ascoldia variegata knipowitschi Soldatov, and Pholidapous dybowskii (Steindachner).

There are four types of antifreeze proteins that are produced by fish per se, and the antifreeze proteins are classified into Type I, Type II, Type III, and AFGP Type. The fish-derived Type I proteins have a molecular weight of about 3.3 kDa to 5.0 kDa, and have an α-helical structure.
The fish-derived Type II proteins have a molecular weight of 14 kDa or less, and have a C-type lectin-like structure with many S-S bonds.
The fish-derived Type III proteins have a molecular weight of about 7 kDa or less, and have a structure characterized by a motif characterized by a two-layered coil structure (also referred to as a two-fold symmetry motif or a pretzel fold).
The antifreeze glycol proteins (AFGP) that are produced by fishes per se have a molecular weight of about 2.2 kDa to 33 kDa, and have a structure consisting of a repeating unit of Ala-Ala-Thr, in which a sugar is bonded at the position of the Thr residue.

Among those described above, Type I antifreeze proteins derived from Pleuronectes pinnifasciatus, Type II antifreeze proteins derived from Hypomesus pretiosus Japonicus (Brovoort), Type III antifreeze proteins derived from Zoarces elongatus Kner, AFGP type antifreeze proteins derived from Eleginus gracilis, and the like may be used as antifreeze proteins that are suitable for sufficiently obtaining the effects of the invention.
So long as the effects of the invention are not impaired, the Type I antifreeze proteins of the invention are not limited to proteins derived from Pleuronectes pinnifasciatus, the Type II antifreeze proteins of the invention are not limited to proteins derived from Hypomesus pretiosus Japonicus (Brovoort), and the Type III antifreeze proteins of the invention are not limited to proteins derived from Zoarces elongatus Kner.

Regarding the antifreeze proteins for the method of increasing thermal hysteresis activity of the invention, products obtained by extraction and purification from fishes, plants, microorganisms, Basidiomycetes, or the like may be used, or products obtained by extraction and purification of antifreeze proteins that have been expressed in cultured cells of a microorganism such as Escherichia coli or insect cells by using a known genetic engineering technique may also be used.

In regard to the method of increasing thermal hysteresis activity of the invention, the concentration of a fish-derived antifreeze protein contained in the mixture is preferably 0.01 mass% to 10 mass%, more preferably 0.05 mass% to 10 mass%, even more preferably 0.1 mass% to 10 mass%, and particularly preferably 0.1 mass% to 5 mass%.
When the concentration is more than or equal to the lower limit of this range, the effects of the invention are sufficiently obtained, and near the upper limit of this concentration range, the effect of increasing the thermal hysteresis activity of the mixture reaches a peak.

The additive for the method of increasing thermal hysteresis activity of the invention is any one or more substances selected from an organic acid, an organic acid salt, an inorganic salt, an amino acid, an amino acid salt, an ammonium salt, a sugar, a sugar alcohol, urea, guanidine hydrochloride, spermidine, spermine, and N,N-dimethylformamide, and is a substance capable of increasing the thermal hysteresis activity of the mixture compared with the thermal hysteresis activity in the case where the mixture contains the antifreeze protein and water only (in the case of not containing the additive). The additives may be used individually, or two or more kinds may be used in combination.

Suitable examples of the organic acid include citric acid, malic acid, maleic acid, tartaric acid, acetic acid, and L-ascorbic acid. The concentration of the organic acid contained in the mixture is preferably 0.1 M to 3 M, more preferably 0.1 M to 1 M, even more preferably 0.5 M to 1 M, and particularly preferably 0.8 M to 1 M. If the concentration is in this range, the effects of the invention are sufficiently obtained.

Suitable examples of the organic acid salt include sodium citrate, trisodium citrate, sodium acetate, sodium lactate, sodium tartrate, disodium succinate, disodium malate, sodium pyruvate, and calcium acetate. The concentration of the organic acid salt contained in the mixture is preferably 0.1 M to 3 M, more preferably 0.1 M to 1 M, even more preferably 0.5 M to 1 M, and particularly preferably 0.8 M to 1 M. If the concentration is in this range, the effects of the invention are sufficiently obtained.

Suitable examples of the inorganic salt include sodium chloride, sodium hydrogen carbonate, sodium carbonate, sodium sulfite, sodium sulfate, sodium thiosulfate, sodium nitrate, sodium dihydrogen phosphate, potassium sulfate, potassium chloride, potassium acetate, potassium carbonate, potassium nitrate, calcium chloride, and calcium nitrate. The concentration of the inorganic salt contained in the mixture is preferably 0.1 M to 3 M, more preferably 0.1 M to 1 M, even more preferably 0.5 M to 1 M, and particularly preferably 0.8 M to 1 M. If the concentration is in this range, the effects of the invention are sufficiently obtained.

Suitable examples of the amino acid or amino acid salt include cystine, asparagine, glutamine, lysine, arginine, arginine hydrochloride, glycine, and histidine. The concentration of the amino acid or amino acid salt contained in the mixture is preferably 0.1 M to 1 M, more preferably 0.5 M to 1 M, and even more preferably 0.8 M to 1 M. If the concentration is in this range, the effects of the invention are sufficiently obtained.

Suitable examples of the ammonium salt include ammonium chloride, ammonium sulfate, ammonium dihydrogen phosphate, triammonium citrate, ammonium iron citrate, ammonium hydrogen carbonate, ammonium alginate, diammonium hydrogen phosphate, diammonium citrate, ammonium sulfite (monohydrate), ammonium thiosulfate, ammonium nitrate, and ammonium acetate. The concentration of the ammonium salt contained in the mixture is preferably 0.01 M to 3 M, more preferably 0.1 M to 1 M, and even more preferably 0.5 M to 1 M. If the concentration is in this range, the effects of the invention are sufficiently obtained.

Suitable examples of the sugar include glucose, fructose, galactose, sucrose, maltose, lactose, trehalose, raffinose, and xylose. The concentration of the sugar contained in the mixture is preferably 0.1 M to 1 M, more preferably 0.2 M to 0.8 M, and even more preferably 0.4 M to 0.6 M. If the concentration is in this range, the effects of the invention are sufficiently obtained.

Suitable examples of the sugar alcohol include sorbitol and glycerin. The concentration of the sugar alcohol contained in the mixture is preferably 0.1 M to 1 M, more preferably 0.2 M to 0.8 M, and even more preferably 0.4 M to 0.6 M. If the concentration is in this range, the effects of the invention are sufficiently obtained.

In the case of using urea as the additive, the concentration of urea contained in the mixture is preferably 0.01 M to 1 M, more preferably 0.1 M to 1 M, and even more preferably 0.5 M to 1 M. If the concentration is in this range, the effects of the invention are sufficiently obtained.
In the case of using guanidine hydrochloride as the additive, the concentration of guanidine hydrochloride contained in the mixture is preferably 0.001 M to 1 M, more preferably 0.01 M to 1 M, and even more preferably 0.1 M to 1 M. If the concentration is in this range, the effects of the invention are sufficiently obtained.

In the case of using spermidine as the additive, the concentration of spermidine contained in the mixture is preferably 0.001 M to 1 M, more preferably 0.01 M to 1 M, and even more preferably 0.1 M to 1 M. If the concentration is in this range, the effects of the invention are sufficiently obtained.
In the case of using spermine as the additive, the concentration of spermine contained in the mixture is preferably 0.001 M to 1 M, more preferably 0.01 M to 1 M, and even more preferably 0.1 M to 1 M. If the concentration is in this range, the effects of the invention are sufficiently obtained.

In the case of using N,N-dimethylformamide as the additive, the concentration of N,N-dimethylformamide in the mixture is preferably 0.1 vol% to 6 vol%, more preferably 2 vol% to 6 vol%, and even more preferably 3 vol% to 6 vol%. If the concentration is in this range, the effects of the invention are sufficiently obtained.

The additives exemplified herein do not cause thermal hysteresis activity by themselves. That is, if the antifreeze protein is not included in the mixture, the thermal hysteresis activity of the mixture does not substantially occur.

Preferred combinations of the antifreeze protein and the additive include the following combinations (A1) to (A5). With these combinations, the thermal hysteresis activity of the mixture can be sufficiently increased.
(A1) For a Type I antifreeze protein derived from Pleuronectes pinnifasciatus, any one or more substances selected from trisodium citrate, sodium acetate, sodium tartrate, disodium succinate, disodium malate, potassium chloride, calcium chloride, sodium hydrogen carbonate, potassium acetate, and potassium carbonate are preferably used as the additive.
(A2) For a Type I antifreeze protein derived from Cottus pollux, sodium chloride is preferably used as the additive.

(A3) For a Type II antifreeze protein derived from Hypomesus pretiosus Japonicus (Brovoort), ammonium sulfate is preferably used as the additive.
(A4) For a Type III antifreeze protein derived from Zoarces elongatus Kner, any one or more substances selected from trisodium citrate, guanidine hydrochloride, arginine, glycine, spermidine, spermine, ammonium chloride, ammonium sulfate, triammonium citrate, ammonium hydrogen carbonate, diammonium hydrogen phosphate, maltose, and raffinose are preferably used as the additive.

(A5) For an AFGP type antifreeze protein derived from Eleginus gracilis, any one or more substances selected from citric acid, malic acid, maleic acid, tartaric acid, trisodium citrate, sodium acetate, sodium lactate, sodium tartrate, disodium succinate, disodium malate, sodium pyruvate, sodium chloride, potassium chloride, calcium chloride, sodium hydrogen carbonate, potassium acetate, potassium carbonate, guanidine hydrochloride, glycine, ammonium chloride, ammonium sulfate, diammonium hydrogen phosphate, diammonium citrate, ammonium sulfite (monohydrate), ammonium thiosulfate, and galactose are preferably used as the additive.

The method of increasing thermal hysteresis activity of the invention has an aspect as a preparation method or a production method of the mixture.
Regarding the method of preparing the mixture in the method of increasing thermal hysteresis activity of the invention, there are no particular limitations as long as the method is one capable of uniformly mixing the antifreeze protein, the additive and water. For example, a method of preparing the mixture as an aqueous solution in which the antifreeze protein and the additive are dissolved in water to the predetermined concentrations may be used. In the aqueous solution, substances other than the antifreeze protein or the additive may also be incorporated.

Examples of the mixture include foods, and preferred examples include ice cream, ice confectionery such as ice candies, and frozen processed foods such as frozen noodles and frozen hamburgers.
When the mixture containing the antifreeze protein, the additive and water of the invention is a food, the growth of ice crystals at the time of cold insulation of the food can be interrupted as the thermal hysteresis activity of the food increases, and recrystallization of ice crystals is suppressed. Therefore, the taste of the food can be made mellow and preferable.

Furthermore, other examples of the mixture include applications other than foods, such as an antifreezing liquid for automobile radiators, a cold insulating agent for cold insulation of fresh foods, and a protective solution for refrigerated storage of microorganisms and cultured cells used in pharmaceutical products and bioresearch applications and the like.

### <Method of reducing thermal deactivation of thermal hysteresis activity>

The method of reducing thermal deactivation of the thermal hysteresis activity as a second embodiment of the invention is a method capable of reducing the extent to which the thermal hysteresis activity exhibited by a composition containing an antifreeze protein and water is decreased after a heat treatment, and is a method of incorporating the antifreeze protein and any one or more substances selected from the following additive group B into the water:
additive group B: an organic acid, an organic acid salt, an inorganic salt, an amino acid, an amino acid salt, an ammonium salt, a sugar, a sugar alcohol, urea, guanidine hydrochloride, acetone, dioxane, 2-chloroethanol, and N,N-dimethylformamide.

That is, the method of reducing the post-heat treatment deactivation of the thermal hysteresis activity exhibited by a composition containing an antifreeze protein and water, which is a second embodiment of the invention, includes incorporating an antifreeze protein and any one or more substances selected from the above additive group B into water, and heating the mixture.

The water may be an aqueous solution or a water-containing material, which contains other substances, as long as the effects of the invention are not impaired. The mixture of the invention is a mixture of water (including the aqueous solution and the water-containing material), the antifreeze protein, and the additive.

The mixture preferably contains water as a main component.

Conventionally, if a mixture that is different from the invention and does not contain an additive is heat treated (for example, for 30 minutes at 40°C to 120°C), the thermal hysteresis activity decreases (this is referred to as thermal deactivation). It can be considered that this is because the antifreeze protein has been denatured by the heat treatment.
However, since the mixture according to the invention contains the additive, even in the case where the mixture is heat treated, a decrease in the thermal hysteresis activity possessed before the heat treatment can be suppressed. That is, the additive for the method of reducing thermal deactivation of the thermal hysteresis activity of the invention offers an effect of imparting heat resistance to the antifreeze protein. This is speculated to be because the additive can reduce the extent to which the antifreeze protein is thermally denatured by the heat treatment.

The antifreeze protein for the method of reducing thermal deactivation of the thermal hysteresis activity of the invention is not particularly limited as long as the antifreeze protein has thermal hysteresis activity, and any known antifreeze protein can be used.

Examples of the antifreeze protein include antifreeze proteins derived from fishes that belong to the genera Myoxocephalus, Gymnocanthus, Hemilepidotus, Furcina, Hypomesus, Spirinchus, Mallotus, Pleurogrammus, Sebastes, Clupea, Limanda, Liopsetta, Clidoderma, Cleisthenes, Microstomus, Lepidopsetta, Platichthys, Kareius, Eopsetta, Gadus, Theragra, Ammodytes, Hypoptychus, Trachurus, Brachyopsis, Pholis, Opisthocentrus, Zoarces, Ascoldia, and Pholidapus.

Specific examples of the antifreeze protein include antifreeze proteins which are collectible from protein collection sources derived from fishes that belong to Myoxocephalus stelleri Tilesius, Gymnocanthus herzensteini Jordan et Starks, Myoxocephalus polyacanthocephalus (Pallas), Hemilepidotus jordani Bean, Furcina osimae Jordan et Starks, Hypomesus pretiosus japonicas (Brevoort)), Hypomesus olidus (Dallas), Spirinchus lanceolatus (Hikita), Mallotus villosus (Muller), Pleurogrammus monopterygius (Pallas), Sebastes steindachneri Hilgendorf, Clupea pallasii Valenciennes, Limanda herzensteini Jordan et Snyder, Limanda punctatissima (Steindachner), Limanda schrenki Schmidt, Limanda aspera (Pallas), Liopsetta obscura (Herzenstein), Clidoderma asperrimum (Temminck et Schlegel), Cleisthenes pinetorumherzensteini (Schmidt), Microctomus achne (Jordan et Starks), Lepidopsetta mochigarei Snyder, Platichthys stellatus (Pallas), Kareius bicoloratus (Basilewsky), Eopsetta grigorjewi (Herzenstein), Gadus macrocephalus Tilesius, Theragra chalcogramma (Pallas), Ammodytes personatus Girard, Hypoptychus dybowskii Steindachner, Trachurus japonicas (Temminck et Schlegel), Brachyopsis, rostratus (Tilesius), Pholis picta (Kner), Opisthocentrus ocellatus (Tilesius), Zoarces elongatus Kner, Ascoldia variegata knipowitschi Soldatov, and Pholidapous dybowskii (Steindachner).

Among those described above, the Type I antifreeze protein derived from Pleuronectes pinnifasciatus, the Type II antifreeze protein derived from Hypomesus pretiosus. Japonicus (Brovoort), the Type III antifreeze protein derived from Zoarces elongatus Kner, the AFGP type antifreeze protein derived from Eleginus gracilis, and the like may be used as antifreeze proteins that are suitable for sufficiently obtaining the effects of the invention.
So long as the effects of the invention are not impaired, the Type I antifreeze proteins of the invention are not limited to proteins derived from Pleuronectes pinnifasciatus, the Type II antifreeze proteins of the invention are not limited to proteins derived from Hypomesus pretiosus Japonicus (Brovoort), and the Type III antifreeze proteins of the invention are not limited to proteins derived from Zoarces elongatus Kner.

Regarding the antifreeze protein according to the second embodiment of the invention, products obtained by extraction and purification from fishes, plants, microorganisms, Basidiomycetes, or the like may be used, or products obtained by extraction and purification of antifreeze proteins that have been expressed in cultured cells of a microorganism such as Escherichia coli or insect cells by using a known genetic engineering technique may also be used.

The concentration of the fish-derived antifreeze protein contained in the mixture in the method of reducing thermal deactivation of the thermal hysteresis activity of the invention is preferably 0.01 mass% to 10 mass%, more preferably 0.05 mass% to 10 mass%, even more preferably 0.1 mass% to 10 mass%, and particularly preferably 0.1 mass% to 5 mass%.
If the concentration is more than or equal to the lower limit of this range, the effects of the invention are sufficiently obtained, and near the upper limit of this concentration range, the effect of increasing the thermal hysteresis activity of the mixture reaches a peak.

The additive for the method of reducing thermal deactivation of the thermal hysteresis activity of the invention is any one or more substances selected from an organic acid, an organic acid salt, an inorganic salt, an amino acid, an amino acid salt, an ammonium salt, a sugar, a sugar alcohol, urea, guanidine hydrochloride, acetone, dioxane, 2-chloroethanol, and N,N-dimethylformamide, and is a substance capable of suppressing a decrease in the thermal hysteresis activity of the mixture that is caused by heating the mixture. The additives described above may be used individually, or two or more kinds may be used in combination.

Suitable examples of the organic acid include citric acid, malic acid, maleic acid, tartaric acid, acetic acid, and L-ascorbic acid. The concentration of the organic acid contained in the mixture is preferably 0.1 M to 3 M, more preferably 0.1 M to 1 M, even more preferably 0.5 M to 1 M, and particularly preferably 0.8 M to 1 M. If the concentration is in this range, the effects of the invention are sufficiently obtained.

Suitable examples of the organic acid salt include sodium citrate, trisodium citrate, sodium acetate, sodium lactate, sodium tartrate, disodium succinate, disodium malate, sodium pyruvate, and calcium acetate. The concentration of the organic acid salt in the mixture is preferably 0.1 M to 3 M, more preferably 0.1 M to 1 M, even more preferably 0.5 M to 1 M, and particularly preferably 0.8 M to 1 M. If the concentration is in this range, the effects of the invention are sufficiently obtained.

Suitable examples of the inorganic salt include sodium chloride, sodium hydrogen carbonate, sodium carbonate, sodium sulfite, sodium sulfate, sodium thiosulfate, sodium nitrate, sodium dihydrogen phosphate, potassium sulfate, potassium chloride, potassium acetate, potassium carbonate, potassium nitrate, calcium chloride, and calcium nitrate. The concentration of the inorganic salt contained in the mixture is preferably 0.1 M to 3 M, more preferably 0.1 M to 1 M, even more preferably 0.5 M to 1 M, and particularly preferably 0.8 M to 1 M. If the concentration is in this range, the effects of the invention are sufficiently obtained.

Suitable examples of the amino acid or amino acid salt include cystine, asparagine, glutamine, lysine, arginine, arginine hydrochloride, glycine, and histidine. The concentration of the amino acid or amino acid salt contained in the mixture is preferably 0.1 M to 1 M, more preferably 0.5 M to 1 M, and even more preferably 0.8 M to 1 M. If the concentration is in this range, the effects of the invention are sufficiently obtained.

Suitable examples of the ammonium salt include ammonium chloride, ammonium sulfate, ammonium dihydrogen phosphate, triammonium citrate, ammonium iron citrate, ammonium hydrogen carbonate, ammonium alginate, diammonium hydrogen phosphate, diammonium citrate, ammonium sulfite (monohydrate), ammonium thiosulfate, ammonium nitrate, and ammonium acetate. The concentration of the ammonium salt contained in the mixture is preferably 0.01 M to 3 M, more preferably 0.1 M to 1 M, and even more preferably 0.5 M to 1 M. If the concentration is in this range, the effects of the invention are sufficiently obtained.

Suitable examples of the sugar include glucose, fructose, galactose, sucrose, maltose, lactose, trehalose, raffinose, and xylose. The concentration of the sugar contained in the mixture is preferably 0.1 M to 1 M, more preferably 0.2 M to 0.8 M and even more preferably 0.4 M to 0.6 M. If the concentration is in this range, the effects of the invention are sufficiently obtained.

Suitable examples of the sugar alcohol include sorbitol and glycerin. The concentration of the sugar alcohol contained in the mixture is preferably 0.1 M to 1 M, more preferably 0.2 M to 0.8 M, and even more preferably 0.4 M to 0.6 M. If the concentration is in this range, the effects of the invention are sufficiently obtained.

In the case of using urea as the additive, the concentration of urea contained in the mixture is preferably 0.01 M to 1 M, more preferably 0.1 M to 1 M, and even more preferably 0.5 M to 1 M.
In the case of using guanidine hydrochloride as the additive, the concentration of guanidine hydrochloride contained in the mixture is preferably 0.001 M to 1 M, more preferably 0.01 M to 1 M, and even more preferably 0.1 M to 1 M. If the concentration is in this range, the effects of the invention are sufficiently obtained.

In the case of using acetone as the additive, the concentration of acetone contained in the mixture is preferably 1 vol% to 25 vol%, more preferably 10 vol% to 25 vol%, and even more preferably 15 vol% to 25 vol%. If the concentration is in this range, the effects of the invention are sufficiently obtained.
In the case of using dioxane (1,4-diethylenedioxane) as the additive, the concentration of dioxane contained in the mixture is preferably 0.1 vol% to 6 vol%, more preferably 2 vol% to 6 vol%, and even more preferably 3 vol% to 6 vol%. If the concentration is in this range, the effects of the invention are sufficiently obtained.
In the case of using 2-chloroethanol (ethylene chlorohydrin) as the additive, the concentration of 2-chloroethanol contained in the mixture is preferably 0.1 vol% to 6 vol%, more preferably 2 vol% to 6 vol%, and even more preferably 3 vol% to 6 vol%. If the concentration is in this range, the effects of the invention are sufficiently obtained.

In the case of using N,N-dimethylformamide as the additive, the concentration of N,N-dimethylformamide contained in the mixture is preferably 0.1 vol% to 6 vol%, more preferably 2 vol% to 6 vol%, and even more preferably 3 vol% to 6 vol%. If the concentration is in this range, the effects of the invention are sufficiently obtained.

The additives exemplified herein do not cause thermal hysteresis activity by themselves. That is, if the antifreeze protein is not included in the mixture, the thermal hysteresis activity of the mixture does not occur.

Preferred combinations of the antifreeze protein and the additive include the following combinations (B1) to (B5). With these combinations, a decrease in the thermal hysteresis activity of the mixture that occurs when the mixture is heated can be sufficiently suppressed.
(B1) For a Type I antifreeze protein derived from Pleuronectes pinnifasciatus, any one or more substances selected from sodium lactate, disodium succinate, disodium malate, sodium chloride, potassium chloride, calcium chloride, sodium hydrogen carbonate, potassium acetate, urea, guanidine hydrochloride, ammonium chloride, ammonium sulfate, diammonium hydrogen phosphate, diammonium citrate, ammonium sulfite (monohydrate), and ammonium thiosulfate are preferably used as the additive.
(B2) For a Type I antifreeze protein derived from Cottus pollux, sodium chloride is preferably used as the additive.

(B3) For a Type **II** antifreeze protein derived from Hypomesus pretiosus Japonicus (Brovoort), ammonium sulfate is preferably used as the additive.
(B4) For a Type **III** antifreeze protein derived from Zoarces elongatus Kner, any one or more substances selected from sodium acetate, urea, guanidine hydrochloride, acetone, dioxane, 2-chloroethanol, N,N-dimethylformamide, asparagine, lysine, arginine, arginine hydrochloride, glycine, ammonium chloride, ammonium sulfate, triammonium citrate, ammonium hydrogen carbonate, diammonium hydrogen phosphate, glucose, fructose, galactose, sucrose, maltose, lactose, trehalose, raffinose, and sorbitol, is preferably used as the additive.

(B5) For an AFGP type antifreeze protein derived from Eleginus gracilis, any one or more substances selected from citric acid, malic acid, sodium lactate, disodium succinate, disodium malate, sodium pyruvate, sodium chloride, potassium chloride, calcium chloride, sodium hydrogen carbonate, potassium acetate, urea, guanidine hydrochloride, glycine, ammonium chloride, ammonium sulfate, diammonium hydrogen phosphate, diammonium citrate, ammonium sulfite (monohydrate), and ammonium thiosulfate are preferably used as the additive.

The method of reducing thermal deactivation of the thermal hysteresis activity of the invention has an aspect as a preparation method or a production method of the mixture.
Regarding the method of preparing the mixture in the method of reducing thermal deactivation of the thermal hysteresis activity of the invention, there are no particular limitations as long as the method is a method capable of uniformly mixing the antifreeze protein, the additive and water. For example, a method of preparing the mixture as an aqueous solution in which the antifreeze protein and the additive are dissolved in water to the predetermined concentrations may be used. In the aqueous solution, substances other than the antifreeze protein or the additive may also be incorporated.

The temperature at the time of heating the mixture may be, for example, in a temperature range of 40°C to 120°C. By heating the mixture to this temperature range, saprophytic bacteria and the like that are contained in the mixture can be sterilized.
In this case, the time of heating the mixture may be set to 1 minute to 30 minutes.
There are no particular limitations on the method of heating the mixture to the temperature range, and a method of placing the mixture in a container, and heating the container by a known method; or a method of irradiating the mixture with microwaves may be used.
Examples of the instances in which such a heat treatment is carried out include cooking and processing, drying, concentration, sterilization and the like of the mixture, as well as the instances in which desired components are dissolved or dispersed in the mixture.

Examples of the mixture in the method of reducing thermal deactivation of the thermal hysteresis activity of the invention include, for example, foods, and preferred examples include ice cream, ice confectionery such as ice candies, and frozen processed foods such as frozen noodles and frozen hamburgers. When the mixture containing the antifreeze protein, the additive and water of the invention is a food, the growth of ice crystals at the time of cold insulation of the food can be interrupted as the thermal hysteresis activity of the food increases, and recrystallization of ice crystals is suppressed. Therefore, the taste of the food can be made mellow and preferable.

Furthermore, other examples of the mixture include applications other than foods, such as an antifreezing liquid for automobile radiators, a cold insulating agent for cold insulation of fresh foods, and a protective solution for refrigerated storage of microorganisms or cultured cells used in pharmaceutical products and bioresearch applications and the like.

### <Composition for increasing thermal hysteresis activity>

The composition for increasing thermal hysteresis activity as a third embodiment of the invention is a composition for increasing thermal hysteresis activity which contains an antifreeze protein, water, and an additive, and is a composition for increasing thermal hysteresis activity containing any one or more substances selected from an organic acid, an organic acid salt, an inorganic salt, an amino acid, an amino acid salt, an ammonium salt, a sugar, a sugar alcohol, urea, guanidine hydrochloride, spermidine, spermine, acetone, dioxane, 2-chloroethanol, and N,N-dimethylformamide in the additive.

The water may be an aqueous solution or a water-containing material, which both contain other substances, as long as the effects of the invention are not impaired. In the aqueous solution, substances other than the antifreeze protein or the additive may also be incorporated.

The composition for increasing thermal hysteresis activity of the invention can further increase the thermal hysteresis activity possessed by an antifreeze protein, or can suppress the decrease caused by heating of the thermal hysteresis activity exhibited by an antifreeze protein.
In the following description, a composition for increasing thermal hysteresis activity of the invention which is capable of enhancing the thermal hysteresis activity possessed by an antifreeze protein will be referred to as a composition for increasing thermal hysteresis activity A.
Furthermore, a composition for increasing thermal hysteresis activity of the invention which is capable of suppressing a decrease caused by heating of the thermal hysteresis activity possessed by an antifreeze protein will be referred to as a composition for increasing thermal hysteresis activity B.

(A) The thermal hysteresis activity exhibited by the composition for increasing thermal hysteresis activity A of the invention primarily depends on the thermal hysteresis activity exhibited by the antifreeze protein; however, by incorporating the additive, the thermal hysteresis activity can be enhanced (promoted), and also, the thermal hysteresis activity can be increased. That is, the additive offers an effect of enhancing (promoting) the thermal hysteresis activity inherently possessed by an antifreeze protein in the composition for increasing thermal hysteresis activity A of the invention.

(B) The thermal hysteresis activity exhibited by the composition for increasing thermal hysteresis activity B of the invention primarily depends on the thermal hysteresis activity exhibited by the antifreeze protein. Here, in the case where an aqueous solution or a water-containing material to which the composition for increasing thermal hysteresis activity B has been added is subjected to a heating treatment (for example, for 30 minutes at 40°C to 120°C), a decrease in the thermal hysteresis activity possessed before the heating treatment can be suppressed. This is speculated to be because the extent to which the thermal hysteresis activity of the antifreeze protein is deteriorated as a result of thermal denaturation can be reduced when the composition for increasing thermal hysteresis activity B contains the additive. That is, the additive offers an effect of imparting heat resistance to the antifreeze protein in the composition for increasing thermal hysteresis activity B of the invention.

The antifreeze protein in the composition for increasing thermal hysteresis activity A or B of the invention is not particularly limited as long as the antifreeze protein has thermal hysteresis activity, and any known antifreeze protein can be used.

Examples of the antifreeze protein include antifreeze proteins derived from fishes that belong to the genera Myoxocephalus, Gymnocanthus, Hemilepidotus, Furcina, Hypomesus, Spirinchus, Mallotus, Pleurogrammus, Sebastes, Clupea, Limanda, Liopsetta, Clidoderma, Cleisthenes, Microstomus, Lepidopsetta, Platichthys, Kareius, Eopsetta, Gadus, Theragra, Ammodytes, Hypoptychus, Trachurus, Brachyopsis, Pholis, Opisthocentrus, Zoarces, Ascoldia, and Pholidapus.

Specifically, examples of the antifreeze protein include antifreeze proteins which are collectible from protein collection sources derived from fishes that belong to Myoxocephalus, stelleri Tilesius, Gymnocanthus herzensteini Jordan et Starks, Myoxocephalus polyacanthocephalus (Pallas), Hemilepidotus jordani Bean, Furcina osimae Jordan et Starks, Hypomesus pretiosus japonicas (Brevoort), Hypomesus olidus (Dallas), Spirinchus lanceolatus (Hikita), Mallotus villosus (Muller), Pleurogrammus monopterygius (Pallas), Sebastes steindachneri Hilgendorf, Clupea pallasii Valenciennes, Limanda herzensteini Jordan et Snyder, Limanda punctatissima (Steindachner), Limanda schrenki Schmidt, Limanda aspera (Pallas), Liopsetta obscura (Herzenstein), Clidoderma asperrimum (Temminck et Schlegel), Cleisthenes pinetorumherzensteini (Schmidt), Microctomus achne (Jordan et Starks), Lepidopsetta mochigarei Snyder, Platichthys stellatus (Pallas), Kareius bicoloratus (Basilewsky), Eopsetta grigorjewi (Herzenstein), Gadus macrocephalus Tilesius, Theragra chalcogramma (Pallas), Ammodytes personatus Girard, Hypoptychus dybowskii Steindachner, Trachurus japonicas (Temminck et Schlegel), Brachyopsis rostratus (Tilesius), Pholis picta (Kner), Opisthocentrus ocellatus (Tilesius), Zoarces elongatus Kner, Ascoldia variegata knipowitschi Soldatov, and Pholidapous dybowskii (Steindachner).

Among those described above, Type I antifreeze proteins derived from Pleuronectes pinnifasciatus, Type II antifreeze proteins derived from Hypomesus pretiosus Japonicus (Brovoort), Type III antifreeze proteins derived from Zoarces elongatus Kner, AFGP type antifreeze proteins derived from Eleginus gracilis, and the like may be used as antifreeze proteins that are suitable for sufficiently obtaining the effects of the invention.
So long as the effects of the invention are not impaired, the Type I antifreeze proteins of the invention are not limited to proteins derived from Pleuronectes pinnifasciatus, the Type II antifreeze proteins of the invention are not limited to proteins derived from Hypomesus pretiosus Japonicus (Brovoort), and the Type III antifreeze proteins of the invention are not limited to proteins derived from Zoarces elongatus Kner.

Regarding the antifreeze proteins in the composition for increasing thermal hysteresis activity A or B of the invention, products obtained by extraction and purification from fishes, plants, microorganisms, Basidiomycetes, or the like may be used, or products obtained by extraction and purification of antifreeze proteins that have been expressed in cultured cells of a microorganism such as Escherichia coli or insect cells by using a known genetic engineering technique may also be used.

The concentration of the fish-derived antifreeze protein contained in the composition for increasing thermal hysteresis activity A or B of the invention is preferably 0.01 mass% to 10 mass%, more preferably 0.05 mass% to 8 mass%, and even more preferably 0.1 mass% to 5 masts%.
When the concentration is more than or equal to the lower limit of this range, the effects of the invention are sufficiently obtained, and near the upper limit of this concentration range, the effect of increasing the thermal hysteresis activity of the composition reaches a peak.

The additive in the composition for increasing thermal hysteresis activity A is any one or more substances selected from an organic acid, an organic acid salt, an inorganic salt, an amino acid, an amino acid salt, an ammonium salt, a sugar, a sugar alcohol, urea, guanidine hydrochloride, spermidine, spermine, and N,N-dimethylformamide, and is a substance which is capable of increasing the thermal hysteresis activity of an aqueous solution or a water-containing material to which a composition for increasing thermal hysteresis activity A has been added, as compared with the thermal hysteresis activity of an aqueous solution or water-containing material to which only the antifreeze protein has been added. The additives may be used individually, or two or more kinds may be used in combination.

The description regarding suitable examples of the additive for the composition for increasing thermal hysteresis activity A is the same as the description listing suitable examples of the additive for the method of increasing thermal hysteresis activity described above.

Furthermore, the description regarding suitable combinations of the antifreeze protein and the additive in the composition for increasing thermal hysteresis activity A is the same as the description listing preferred combinations for the method of increasing thermal hysteresis activity mentioned above.

The additive for the composition for increasing thermal hysteresis activity B is any one or more substances selected from an organic acid, an organic acid salt, an inorganic salt, an amino acid, an amino acid salt, an ammonium salt, a sugar, a sugar alcohol, urea, guanidine hydrochloride, acetone, dioxane, 2-chloroethanol, and N,N-dimethylformamide, and when an aqueous solution or a water-containing material, to which the composition for increasing thermal hysteresis activity B has been added, is subjected to a heating treatment (for example, for 30 minutes at 40°C to 120°C), a decrease in the thermal hysteresis activity possessed before the heating treatment can be suppressed. The additives described above may be used individually, or two or more kinds may also be used in combination.

The description regarding suitable examples of the additive for the composition for increasing thermal hysteresis activity B is the same as the description listing suitable examples of the additive for the method of reducing thermal deactivation of the thermal hysteresis activity described above.

Furthermore, the description regarding preferred combinations of the antifreeze protein and the additive for the composition for increasing thermal hysteresis activity B is the same as the description listing preferred examples of the combination for the method of reducing thermal deactivation of the thermal hysteresis activity.

The concentration of the organic acid contained in the composition is preferably 0.1 M to 3 M, more preferably 0.1 M to 1 M, even more preferably 0.5 M to 1 M, and particularly preferably 0.8 M to 1 M.
The concentration of the organic acid salt contained in the composition is preferably 0.1 M to 3 M, more preferably 0.1 M to 1 M, even more preferably 0.5 M to 1 M, and particularly preferably 0.8 M to 1 M.
The concentration of the inorganic salt contained in the composition is preferably 0.1 M to 3 M, more preferably 0.1 M to 1 M, even more preferably 0.5 M to 1 M, and particularly preferably 0.8 M to 1 M.
The concentration of the amino acid or amino acid salt contained in the composition is preferably 0.1 M to 1 M, more preferably 0.5 M to 1 M, and even more preferably 0.8 M to 1 M.
The concentration of the ammonium salt contained in the composition is preferably 0.01 M to 3 M, more preferably 0.1 M to 1 M, and even more preferably 0.5 M to 1 M.
The concentration of the sugar contained in the composition is preferably 0.1 M to 1 M, more preferably 0.2 M to 0.8 M, and even more preferably 0.4 M to 0.6 M.
The concentration of the sugar alcohol contained in the composition is preferably 0.1 M to 1 M, more preferably 0.2 M to 0.8 M, and even more preferably 0.4 M to 0.6 M.
When the concentrations of the aforementioned additives are in the ranges described above, the effects of the invention are sufficiently obtained.

The concentration of urea in the composition is preferably 0.01 M to 1 M, more preferably 0.1 M to 1 M, and even more preferably 0.5 M to 1 M.
The concentration of guanidine hydrochloride contained in the composition is preferably 0.001 M to 1 M, more preferably 0.01 M to 1 M, and even more preferably 0.1 M to 1 M.

The concentration of acetone contained in the composition is preferably 1 vol% to 25 vol%, more preferably 10 vol% to 25 vol%, and even more preferably 15 vol% to 25 vol%.
The concentration of dioxane (1,4-diethylenedioxane) contained in the composition is preferably 0.1 vol% to 6 vol%, more preferably 2 vol% to 6 vol%, and even more preferably 3 vol% to 6 vol%.
The concentration of 2-chloroethanol (ethylene chlorohydrin) contained in the composition is preferably 0.1 vol% to 6 vol%, more preferably 2 vol% to 6 vol%, and even more preferably 3 vol% to 6 vol%.
When the concentrations of the aforementioned additives are in the ranges described above, the effects of the invention are sufficiently obtained.

The concentration of spermidine contained in the composition is preferably 0.001 M to 1 M, more preferably 0.01 M to 1 M, and even more preferably 0.1 M to 1 M.
The concentration of spermine contained in the composition is preferably 0.001 M to 1 M, more preferably 0.01 M to 1 M, and even more preferably 0.1 M to 1 M.
The concentration of N,N-dimethylformamide contained in the composition is preferably 0.1 vol% to 6 vol%, more preferably 2 vol% to 6 vol%, and even more preferably 3 vol% to 6 vol%.
When the concentrations of the aforementioned additives are in the ranges described above, the effects of the invention are sufficiently obtained.

The composition for increasing thermal hysteresis activity A, and the composition for increasing thermal hysteresis activity B can be produced by, for example, mixing a powder obtained by freeze-drying the antifreeze protein that has been extracted and purified by a known method, with the additive and water at a predetermined proportion.
So long as the effects of the invention are not impaired, there are no particular limitations on the method of mixing, the order of mixing, and the like of the antifreeze protein, the additive and the water.

The composition for increasing thermal hysteresis activity of the invention that is in a powder form can increase the thermal hysteresis activity of water or a water-containing material by a predetermined amount of the composition being uniformly mixed with the water or the water-containing material.

More specifically, when the composition for increasing thermal hysteresis activity is incorporated into ice cream, ice confectionery such as ice candies, or frozen processed foods such as frozen noodles and frozen hamburgers, the growth of ice crystals at the time of refrigeration of the foods can be interrupted as the thermal hysteresis activity of these foods increases. Furthermore, since recrystallization of ice crystals is suppressed, the taste of the food can be made mellow and preferable. Also, the composition for increasing thermal hysteresis activity can also be used by incorporating the composition into products other than foods, such as an antifreezing liquid for automobile radiators, a cold insulating agent for cold insulation of fresh foods, and a protective solution for refrigerated storage of microorganisms and cultured cells used in pharmaceutical products and bioresearch applications and the like.

### Examples

Next, the invention will be described in more detail with reference to Examples, but the invention is not intended to be limited to these examples.

### (Preparation of fish-derived antifreeze proteins)

The fish-derived antifreeze proteins used in the following Examples were obtained from the four kinds of fish indicated in Table 1, which were caught by Notsuke Fisheries Cooperative Association of Betsukai-cho, Notsuke-gun, Hokkaido, through extraction and purification.

The methods for extraction and purification of the antifreeze protein are as follows.
First, a fish raw material and deionized water in equal amounts by mass were pulverized and mixed in a mixer, and then the suspension thus obtained was separated into a supernatant and a precipitate by centrifugation. The supernatant thus obtained was passed through a precision filter membrane having a pore size of 100 µm, and thus foreign materials included in the supernatant were removed.

Next, single-time ultrafiltration was carried out by using an ultrafiltration membrane having a molecular weight cut-off of 30,000 (Da) for the Type I AFP, the Type II AFP, and the AFGP, and by using an ultrafiltration membrane having a molecular weight cut-off of 10,000 (Da) for the Type III AFP. Thereby, foreign proteins having molecular weights larger than the relevant antifreeze proteins included in the supernatant were removed, and thus a liquid of the relevant antifreeze protein (membrane-permeated liquid) was obtained. Subsequently, second-round ultrafiltration was carried out by using an ultrafiltration membrane having a molecular weight cut-off of 3,000 (Da) for all of the AFP's, and thereby foreign proteins having molecular weights smaller than the relevant antifreeze proteins included in the liquid were removed. Thus, a concentrated liquid of the relevant antifreeze proteins (liquid that did not pass through the ultrafiltration membrane) was obtained.

**[Table 1]**

| | AFP Type (kind) |
|---|---|
| Pleuronectes pinnifasciatus-derived antifreeze protein | Type I |
| Hvpomesus pretiosus Japonicus (Brovoort) -derived antifreeze protein | Type II |
| Zoarces elongatus Kner-derived antifreeze protein | Type III |
| Elegjnus gracilis-derived antifreeze protein | AFGP |

### (Preparation of sample for measuring thermal hysteresis activity)

In Examples 1 to 92 and Comparative Examples 1 to 4, an antifreeze protein, an additive and deionized water were mixed such that the final concentration of the purified antifreeze protein would be 5 mass% (50 mg/ml), and the final concentration of the additive would be the concentrations indicated in Tables 2 to 9, and thus samples for measuring thermal hysteresis activity were obtained.

In Examples 93 to 314, an antifreeze protein, an additive and deionized water were mixed such that the final concentration of the purified antifreeze protein and the final concentration of the additive would be concentrations indicated in Tables 10 to 19, and thus samples for measuring thermal hysteresis activity were obtained.

Regarding the method of preparing the samples, when the final concentration of the additive in each of the samples was 1/2 or less of the saturation concentration of the additive, a method of mixing equal amounts of an aqueous solution prepared by dissolving the additive at a concentration of two times the final concentration, and the antifreeze protein at 10 mass%, was used. Furthermore, when the final concentration of the additive in each of the samples was 1/2 or more of the saturation concentration of the additive, an aqueous solution prepared by dissolving the additive at a saturation concentration was mixed with the antifreeze protein at 10 mass%, subsequently deionized water was added thereto, and the mixture was diluted to obtain a predetermined additive concentration. Each of the samples was uniformly mixed, and then was centrifuged at 10,000 rpm (12,000 G) for 10 minutes at 4°C. Thus, a sample from which insoluble components were removed was used as a sample (I) for measuring thermal hysteresis activity.

### (Method of measuring thermal hysteresis activity (°C))

The thermal hysteresis activity of the samples was measured by the following Steps 1 to 3, by using a microscope (BX51; Olympus Corp.) equipped with an apparatus having a temperature control mechanism for enabling the observation of the morphology of ice crystals in the sample (LK-600PM; Linkam Scientific Instruments, Ltd.).

Step 1: 1 µl of a sample was dropped on a circular cover glass mounted on the microscope stage, and then the sample was cooled at a rate of 50°C per minute, to a temperature at which the entire sample would freeze (see FIG. 1).
Step 2: After freezing of the sample was confirmed, the temperature was elevated at a rate of 10°C per minute to a temperature close to the melting point of the sample, and while caution was taken not to allow ice crystals in the sample to melt completely, the stage temperature was controlled. When the ice crystals in the field of vision melted and decreased in number, the rate of temperature increase was changed to about 1°C to 5°C per minute, and the number of ice crystals in the field of vision was set to 5 or less. Thereafter, the temperature of the stage was strictly controlled at a rate of about 0.5°C to 5°C per minute, and the temperature at the initiation of melting of residual ice crystals was determined as the melting point (up to the second digit after the decimal point).
Step 3: When the melting point was determined in Step 2, while the system was cooled at a rate of about 0.2°C to 1°C per minute, the temperature at which significant growth of residual ice crystals was initiated (freezing point) was determined.

In Step 3, if the stage temperature was lower than the temperature range in which the antifreeze protein in the sample could suppress the growth of ice crystals, ice crystals could start to rapidly grow. The temperature difference between the temperature at this time and the melting point was measured as the thermal hysteresis activity (°C). The results are described in Tables 3 to 19.

### (Method of measuring thermal hysteresis activity h (°C))

The sample (I) for measuring thermal hysteresis activity thus prepared was heated to 90°C for 30 minutes by using a water bath, and then was centrifuged at 10,000 rpm (12,000 G) for 10 minutes at 4°C. The sample from which insoluble components were removed was used as a sample (II) for measuring thermal hysteresis activity.
The thermal hysteresis activity h (°C) of the sample (II) thus obtained was measured by the same method as that used for the measurement of the thermal hysteresis activity (°C). The results are described in Tables 3 to 9.

Furthermore, as a Comparative Example, an antifreeze protein and deionized water only were mixed such that the final concentration of the antifreeze protein would be 5 mass% (50 mg/ml), and thus the mixture was used as a sample (III) for measuring thermal hysteresis activity.
The thermal hysteresis activity (°C) of the sample (III) thus obtained, and the thermal hysteresis activity h (°C) were measured by the same method as described above. The results are shown in Table 2.

**[Table 2]**

| | Additive | AFP type (kind) | Additive concentration (M) | Thermal hysteresis activity (°C) | Thermal hysteresis activity h(°C) |
|---|---|---|---|---|---|
| Comparative Example 1 | None | Type I AFP | 0 | 1.5-1.7 | 0.63-0.71 |
| Comparative Example 2 | None | Type I AFP | 0 | 0.32 | 0.17 |
| Comparative Example 3 | None | Type I AFP | 0 | 1.3 | 0.45-0.62 |
| Comparative Example 4 | None | AFGP | 0 | 0.7-0.9 | 0.7-0.77 |

**[Table 3]**

| | Additive | AFP type (kind) | Additive concentration (M) | Thermal hysteresis activity (°C) | Thermal hysteresis activity h (°C) | Promotion effect | Heat resistance effect |
|---|---|---|---|---|---|---|---|
| Example 1 | Sodium citrate | AFGP | 1.0 | 1.51 | 1.08 | A | B |
| Example 2 | Malic acid | AFGP | 1.0 | 1.03 | 1.04 | B | B |
| Example 3 | Maleic acid | AFGP | 1.0 | 1.04 | - | B | - |
| Example 4 | Tartaric acid | AFGP | 1.0 | 1.41 | - | A | - |
| Example 5 | Trisodium citrate | Type I AFP | 1.0 | 2.64 | - | A | - |
| Example 6 | Trisodium citrate | Type III AFP | 0.5 | 2.14 | - | A | - |
| Example 7 | Trisodium citrate | AFGP | 1.0 | 5.55 | - | A | - |
| Example 8 | Sodium acetate | Type I AFP | 1.0 | 2.45 | - | A | - |
| Example 9 | Sodium acetate | Type III AFP | 0.5 | 1.76 | 0.86 | B | A |
| Example 10 | Sodium acetate | AFGP | 1.0 | 1.88 | - | A | - |
| Example 11 | Sodium lactate | Type I AFP | 1.0 | 1.68 | 1.09 | B | A |
| Example 12 | Sodium lactate | AFGP | 1.0 | 2.06 | 1.84 | A | A |
| Example 13 | Sodium tartrate | Type I AFP | 1.0 | 4.50 | - | A | - |
| Example 14 | Sodium tartrate | AFGP | 1.0 | 3.56 | - | A | - |
| Example 15 | Disodium succinate | Type I AFP | 1.0 | 3.84 | 1.5 | A | A |
| Example 16 | Disodium succinate | AFGP | 1.0 | 5.82 | 2.05 | A | A |
| Example 17 | Disodium malate | Type I AFP | 1.0 | 3.92 | 1.33 | A | A |
| Example 18 | Disodium malate | AFGP | 1.0 | 4.90 | 2.42 | A | A |
| Example 19 | Sodium pyruvate | AFGP | 1.0 | 1.25 | 0.8 | A | B |

**[Table 4]**

| | Additive | AFP type (kind) | Additive concentration (M) | Thermal hysteresis activity (°C) | Thermal hysteresis activity h (°C) | Promotion effect | Heat resistance effect |
|---|---|---|---|---|---|---|---|
| Example 20 | Sodium chloride | Type I AFP | 1.0 | 1.79 | 0.96 | B | B |
| Example 21 | Sodium chloride | AFGP | 1.0 | 1.27 | 1.29 | A | A |
| Example 22 | Potassium chloride | Type I AFP | 1.0 | 2.33 | 0.95 | B | B |
| Example 23 | Potassium chloride | AFGP | 1.0 | 1.17 | 1.09 | B | B |
| Example 24 | Calcium chloride | Type I AFP | 1.0 | 2.67 | 1.36 | A | A |
| Example 25 | Calcium chloride | AFGP | 1.0 | 1.21 | 1.17 | A | A |
| Example 26 | Sodium hydrogen carbonate | Type I AFP | 1.0 | 3.56 | 1.36 | A | A |
| Example 27 | Sodium hydrogen carbonate | AFGP | 1.0 | 2.23 | 1.68 | A | A |
| Example 28 | Potassium acetate | Type I AFP | 1.0 | 2.98 | 0.89 | A | B |
| Example 29 | Potassium acetate | AFGP | 1.0 | 1.70 | 1.39 | A | A |
| Example 30 | Potassium carbonate | Type I AFP | 1.0 | 2.67 | 0.75 | A | B |
| Example 31 | Potassium carbonate | AFGP | 1.0 | 2.91 | - | A | - |

**[Table 5]**

| | Additive | AFP type (kind) | Additive concentration | Thermal hysteresis activity (°C) | Thermal hysteresis activity h(°C) | Promotion effect | Heat resistance effect |
|---|---|---|---|---|---|---|---|
| Example 32 | Urea | Type I AFP | 1.0 (M) | 1.20 | 0.93 | C | B |
| Example 33 | Urea | Type III AFP | 0.1 (M) | 1.15 | 0.61 | C | B |
| Example 34 | Urea | Type III AFP | 1.0 (M) | 1.40 | 1.00 | B | A |
| Example 35 | Urea | AFGP | 1.0 (M) | 1.00 | 1.02 | B | B |
| Example 36 | Guanidine hydrochloride | Type I AFP | 1.0 (M) | 1.48 | 1.37 | c | A |
| Example 37 | Guanidine hydrochloride | Type III AFP | 0.1 (M) | 1.65 | 0.85 | B | A |
| Example 38 | Guanidine hydrochloride | Type III AFP | 1.0 (M) | 1.90 | 0.85 | B | A |
| Example 39 | Guanidine hydrochloride | AFGP | 1.0 (M) | 1.22 | 1.08 | A | B |
| Example 40 | Acetone | Type III AFP | 20(vol%) | 0.70 | 0.75 | C | B |
| Example 41 | Dioxane | Type III AFP | 5 (vol%) | 0.80 | 0.65 | C | B |
| Example 42 | 2-Chloroethanol | Type III AFP | 5 (vol%) | 1.10 | 0.65 | C | B |
| Example 43 | N,N-dimethylformamide | Type III AFP | 5 (vol%) | 1.30 | 0.90 | C | A |

**[Table 6]**

| | Additive | AFP type (kind) | Additive concentration (M) | Thermal hysteresis activity (°C) | Thermal hysteresis activity h(°C) | Promotion effect | Heat resistance effect |
|---|---|---|---|---|---|---|---|
| Example 44 | Asparagine | Type III AFP | 0.1 | 1.40 | 0.75 | B | B |
| Example 45 | Asparagine | Type III AFP | 0.5 | 1.40 | 0.75 | B | B |
| Example 46 | Lysine | Type III AFP | 0.01 | 1.20 | 0.80 | C | B |
| Example 47 | Lysine | Type III AFP | 0.1 | 1.55 | 0.80 | B | B |
| Example 48 | Lysine | Type III AFP | 0.5 | 1.55 | 0.90 | B | A |
| Example 49 | Arginine | Type III AFP | 0.001 | 1.37 | 0.68 | B | B |
| Example 50 | Arginine | Type III AFP | 0.01 | 1.26 | 0.80 | C | A |
| Example 51 | Arginine | Type III AFP | 0.1 | 1.42 | 0.66 | B | B |
| Example 52 | Arginine | Type III AFP | 0.5 | 1.73 | 0.74 | B | B |
| Example 53 | Arginine hydrochloride | Type III AFP | 0.5 | 2.35 | 1.18 | A | A |
| Example 54 | Glycine | Type III AFP | 0.001 | 1.45 | 0.76 | B | B |
| Example 55 | Glycine | Type III AFP | 0.01 | 1.40 | 0.63 | B | B |
| Example 56 | Glycine | Type III AFP | 0.1 | 1.30 | 0.75 | C | B |
| Example 57 | Glycine | Type III AFP | 0.5 | 1.49 | 1.23 | B | A |
| Example 58 | Glycine | AFGP | 1.0 | 1.53 | 1.38 | A | A |
| Example 59 | Spermidine | Type III AFP | 1.0 | 2.30 | - | A | - |
| Example 60 | Spermine | Type III AFP | 1.0 | 1.96 | - | A | - |

**[Table 7]**

| | Additive | AFP type (kind) | Additive concentration (M) | Thermal hysteresis activity (°C) | Thermal hysteresis activity (°C) | Promotion effect | Heat resistance effect |
|---|---|---|---|---|---|---|---|
| Example 61 | Ammonium chloride | Type I AFP | 1.0 | 2.09 | 1.09 | B | A |
| Example 62 | Ammonium chloride | Type III AFP | 0.1 | 1.15 | 0.71 | C | B |
| Example 63 | Ammonium chloride | Type III AFP | 1.0 | 1.90 | 1.57 | B | A |
| Example 64 | Ammonium chloride | AFGP | 1.0 | 1.17 | 1.20 | B | A |
| Example 65 | Ammonium sulfate | Type I AFP | 1.0 | 1.39 | 1.28 | C | A |
| Example 66 | Ammonium sulfate | Type II AFP | 1.0 | 0.53 | 0.25 | A | B |
| Example 67 | Ammonium sulfate | Type III AFP | 0.01 | 1.14 | 0.57 | C | B |
| Example 68 | Ammonium sulfate | Type III AFP | 0.1 | 1.16 | 0.66 | C | B |
| Example 69 | Ammonium sulfate | Type III AFP | 1.0 | 2.68 | 0.96 | A | A |
| Example 70 | Ammonium sulfate | AFGP | 1.0 | 3.17 | 2.63 | A | A |
| Example 71 | Triammonium citrate | Type III AFP | 0.5 | 2.18 | 0.80 | A | B |
| Example 72 | Ammonium hydrogen carbonate | Type III AFP | 0.5 | 1.52 | 0.78 | B | B |

**[Table 8]**

| | Additive | AFP type (kind) | Additive concentration (M) | Thermal hysteresis activity (°C) | Thermal hysteresis activity h (°C) | Promotion effect | Heat resistance effect |
|---|---|---|---|---|---|---|---|
| Example 73 | Diammonium hydrogen phosphate | Type I AFP | 1.0 | 2.07 | 1.68 | B | A |
| Example 74 | Diammonium hydrogen phosphate | Type III AFP | 0.1 | 1.71 | 0.60 | B | B |
| Example 75 | Diammonium hydrogen phosphate | Type III AFP | 1.0 | - | 0.88 | - | A |
| Example 76 | Diammonium hydrogen phosphate | AFGP | 1.0 | 2.93 | 2.30 | A | A |
| Example 77 | Diammonium citrate | Type I AFP | 1.0 | 1.86 | 1.43 | B | A |
| Example 78 | Diammonium citrate | AFGP | 1.0 | 3.43 | 2.86 | A | A |
| Example 79 | Ammonium sulfite (monohydrate) | Type I AFP | 1.0 | 1.42 | 1.73 | C | A |
| Example 80 | Ammonium sulfite (monohydrate) | AFGP | 1.0 | 2.69 | 2.35 | A | A |
| Example 81 | Ammonium thiosulfate | Type I AFP | 1.0 | 1.44 | 1.59 | c | A |
| Example 82 | Ammonium thiosulfate | AFGP | 1.0 | 3.39 | 3.22 | A | A |

**[Table 9]**

| | Additive | AFP type (kind) | Additive concentration (M) | Thermal hysteresis activity (°C) | Thermal hysteresis activity h (°C) | Promotion effect | Heat resistance effect |
|---|---|---|---|---|---|---|---|
| Example 83 | Glucose | Type III AFP | 0.5 | 1.34 | 0.82 | B | A |
| Example 84 | Fructose | Type III AFP | 0.5 | 1.38 | 0.77 | B | B |
| Example 85 | Galactose | Type III AFP | 0.5 | 1.37 | 0.84 | B | A |
| Example 86 | Sorbitol | Type III AFP | 0.5 | 1.34 | 0.82 | B | A |
| Example 87 | Galactose | AFGP | 30 | 2.70 | - | A | - |
| Example 88 | Sucrose | Type III AFP | 0.5 | 1.73 | 0.87 | B | A |
| Example 89 | Maltose | Type III AFP | 0.5 | 2.25 | 0.92 | A | A |
| Example 90 | Lactose | Type III AFP | 0.5 | 1.70 | 0.88 | B | A |
| Example 91 | Trehalose | Type III AFP | 0.5 | 1.73 | 0.98 | B | A |
| Example 92 | Raffinose | Type III AFP | 0.5 | 1.60 | 0.86 | B | A |

**[Table 10]**

| Example | Additive | AFP type (kind) | AFP concentration (mass%) | Additive concentration, (M) | Thermal hysteresis activity (°C) | Thermal hysteresis activity h (°C) | Promotion effect | Heat resistance effect |
|---|---|---|---|---|---|---|---|---|
| Example 93 | Acetic acid | Type I AFP | 0.1 | 0.5 | 0.15 | - | A | - |
| Example 94 | Acetic acid | Type I AFP | 0.5 | 0.5 | 0.26 | - | B | - |
| Example 95 | L-ascorbic acid | Type I AFP | 0.1 | 0.5 | 0.13 | - | A | - |
| Example 96 | L-ascorbic acid | Type I AFP | 0.5 | 0.5 | 0.23 | 0.22 | C | B |
| Example 97 | Citric acid | Type I AFP | 0.1 | 0.5 | 0.15 | - | A | - |
| Example 98 | Citric acid | Type I AFP | 0.5 | 0.5 | 0.25 | - | C | - |
| Example 99 | Maleic acid | Type I AFP | 0.1 | 0.5 | 0.17 | - | A | - |
| Example 100 | Maleic acid | Type I AFP | 0.5 | 0.5 | 0.17 | - | C | - |
| Example 101 | Tartaric acid | Type I AFP | 0.1 | 0.5 | 0.10 | - | A | - |
| Example 102 | Tartaric acid | Type I AFP | 0.5 | 0.5 | 0.21 | - | C | - |
| Example 103 | Trisodium citrate | Type I AFP | 0.01 | 0.5 | 0.21 | 0.22 | A | A |
| Example 104 | Trisodium citrate | Type I AFP | 0.1 | 0.5 | 0.52 | - | A | - |
| Example 105 | Trisodium citrate | Type I AFP | 0.5 | 0.5 | 1.16 | - | A | - |
| Example 106 | Sodium acetate | Type I AFP | 0.01 | 0.5 | 0.10 | - | A | - |
| Example 107 | Sodium acetate | Type I AFP | 0.1 | 0.5 | 0.23 | - | A | - |
| Example 108 | Sodium acetate | Type I AFP | 0.5 | 0.5 | 0.40 | - | A | - |
| Example 109 | Sodium pyruvate | Type I AFP | 0.1 | 0.5 | 0.23 | - | A | - |
| Example 110 | Sodium pyruvate | Type I AFP | 0.5 | 0.5 | 0.38 | - | A | - |
| Example 111 | Calcium acetate | Type I AFP | 0.1 | 0.5 | 0.30 | - | A | - |
| Example 112 | Calcium acetate | Type I AFP | 0.5 | 0.5 | 0.46 | 0.44 | A | A |
| Example 113 | Sodium chloride | Type I AFP | 0.1 | 0.5 | 0.20 | - | A | - |
| Example 114 | Sodium chloride | Type I AFP | 0.5 | 0.5 | 0.37 | - | B | - |
| Example 115 | Sodium hydrogen carbonate | Type I AFP | 0.1 | 0.5 | 0.21 | - | A | - |
| Example 116 | Sodium hydrogen carbonate | Type I AFP | 0.5 | 0.5 | 0.38 | - | A | - |
| Example 117 | Sodium carbonate | Type I AFP | 0.1 | 0.5 | 0.35 | - | A | - |
| Example 118 | Sodiumcarbonate | Type I AFP | 0.5 | 0.5 | 0.63 | - | A | - |

**[Table 11]**

| Example | Additive | AFP type (kind) | AFP concentration (mass%) | Additive concentration (M) | Thermal hysteresis activity (°C) | Thermal hysteresis activity h (°C) | Promotion effect | Heat resistance effect |
|---|---|---|---|---|---|---|---|---|
| Example 119 | Sodium sulfite | Type I AFP | 0.01 | 0.5 | 0.17 | - | A | - |
| Example 120 | Sodium sulfite | Type I AFP | 0.1 | 0.5 | 0.34 | - | A | - |
| Example 121 | Sodium sulfite | Type I AFP | 0.5 | 0.5 | 0.62 | 0.68 | A | A |
| Example 122 | Sodium thiosulfate | Type I AFP | 0.1 | 0.5 | 0.35 | - | A | - |
| Example 123 | Sodium thiosulfate | Type I AFP | 0.5 | 0.5 | 0.70 | - | A | - |
| Example 124 | Sodium nitrate | Type I AFP | 0.1 | 0.5 | 0.20 | - | A | - |
| Example 125 | Sodium nitrate | Type I AFP | 0.5 | 0.5 | 0.38 | - | A | - |
| Example 126 | Sodium dihydrogen phosphate | Type I AFP | 0.1 | 0.5 | 0.25 | - | A | - |
| Example 127 | Sodium dihydrogen phosphate | Type I AFP | 0.5 | 0.5 | 0.51 | - | A | - |
| Example 128 | Potassium chloride | Type I AFP | 0.1 | 0.5 | 0.22 | - | A | - |
| Example 129 | Potassium chloride | Type I AFP | 0.5 | 0.5 | 0.38 | - | A | - |
| Example 130 | Potassium acetate | Type I AFP | 0.1 | 0.5 | 0.18 | - | A | - |
| Example 131 | Potassium acetate | Type I AFP | 0.5 | 0.5 | 0.35 | - | B | - |
| Example 132 | Potassium carbonate | Type I AFP | 0.1 | 0.5 | 0.28 | - | A | - |
| Example 133 | Potassium carbonate | Type I AFP | 0.5 | 0.5 | 0.89 | - | A | - |
| Example 134 | Potassium nitrate | Type I AFP | 0.1 | 0.5 | 0.19 | - | A | |
| Example 135 | Potassium nitrate | Type I AFP | 0.5 | 0.5 | 0.35 | - | B | |
| Example 136 | Calcium chloride | Type I AFP | 0.1 | 0.5 | 0.29 | - | A | - |
| Example 137 | Calcium chloride | Type I AFP | 0.5 | 0.5 | 0.40 | - | A | - |
| Example 138 | Calcium nitrate | Type I AFP | 0.1 | 0.5 | 0.18 | - | A | - |
| Example 139 | Calcium nitrate | Type I AFP | 0.5 | 0.5 | 0.46 | - | A | - |
| Example 140 | Ammonium chloride | Type I AFP | 0.1 | 0.5 | 0.31 | - | A | - |
| Example 141 | Ammonium chloride | Type I AFP | 0.5 | 0.5 | 0.53 | - | A | - |
| Example 142 | Ammonium sulfate | Type I AFP | 0.1 | 0.5 | 0.54 | - | A | - |
| Example 143 | Ammonium sulfate | Type I AFP | 0.5 | 0.5 | 0.93 | - | A | - |

**[Table 12]**

| Example | Additive | AFP type (kind) | AFP concentration (mass%) | Additive concentration (M) | Thermal hysteresis activity (°C) | Thermal hysteresis activity h (°C) | Promotion effect | Heat resistance effect |
|---|---|---|---|---|---|---|---|---|
| Example 144 | Ammonium nitrate | Type I AFP | 0.1 | 0.5 | 0.38 | - | A | - |
| Example 145 | Ammonium nitrate | Type I AFP | 0.5 | 0.5 | 0.56 | - | A | - |
| Example 146 | Ammonium acetate | Type I AFP | 0.1 | 0.5 | 0.39 | - | A | - |
| Example 147 | Ammonium acetate | Type I AFP | 0.5 | 0.5 | 0.70 | 0.60 | A | A |
| Example 148 | Ammonium dihydrogen phosphate | Type I AFP | 0.1 | 0.5 | 0.47 | - | A | - |
| Example 149 | Ammonium dihydrogen phosphate | Type I AFP | 0.5 | 0.5 | 0.63 | - | A | - |
| Example 150 | Triammonium citrate | Type I AFP | 0.1 | 0.5 | 0.70 | - | A | - |
| Example 151 | Triammonium citrate | Type I AFP | 0.5 | 0.5 | 1.16 | - | A | - |
| Example 152 | Diammonium citrate | Type I AFP | 0.5 | 0.5 | 0.85 | - | A | - |
| Example 153 | Diammonium hydrogen phosphate | Type I AFP | 0.1 | 0.5 | 0.43 | - | A | - |
| Example 154 | Diammonium hydrogen phosphate | Type I AFP | 0.5 | 0.5 | 0.79 | - | A | - |
| Example 155 | Ammonium sulfite (monohydrate) | Type I AFP | 0.1 | 0.5 | 0.45 | - | A | - |
| Example 156 | Ammmonium sulfite (monohydrate) | Type I AFP | 0.5 | 0.5 | 0.82 | - | A | - |
| Example 157 | Ammonium thiosulfate | Type I AFP | 0.1 | 0.5 | 0.47 | - | A | - |
| Example 158 | Ammonium thiosulfate | Type I AFP | 0.5 | 0.5 | 0.80 | - | A | - |

**[Table 13]**

| Example | Additive | AFP type (kind) | AFP concentration (mass%) | Additive concentration (M) | Thermal hysteresis activity (°C) | Thermal hysteresis activity h(°C) | Promotion effect | Heat resistance effect |
|---|---|---|---|---|---|---|---|---|
| Example 159 | Citric acid | Type III AFP | 0.5 | 0.5 | 0.19 | - | B | - |
| Example 160 | L-ascorbic acid | Type III AFP | 0.5 | 0.5 | 0.21 | 0.21 | A | A |
| Example 161 | Citric acid | Type III AFP | 0.5 | 0.5 | 0.22 | - | A | - |
| Example 162 | Maleic acid | Type III AFP | 0.5 | 0.5 | 0.19 | - | B | - |
| Example 163 | Tartaric acid | Type III AFP | 0.5 | 0.5 | 0.22 | - | A | - |
| Example 164 | Trisodium citrate | Type III AFP | 0.1 | 0.5 | 0.30 | - | A | - |
| Example 165 | Trisodium citrate | Type III AFP | 0.5 | 0.5 | 0.51 | - | A | - |
| Example 166 | Sodium acetate | Type III AFP | 0.1 | 0.5 | 0.31 | - | A | - |
| Example 167 | Sodium acetate | Type III AFP | 0.5 | 0.5 | 0.30 | - | A | - |
| Example 168 | Sodium pyruvate | Type III AFP | 0.1 | 0.5 | 0.18 | - | A | - |
| Example 169 | Sodium pyruvate | Type III AFP | 0.5 | 0.5 | 0.35 | - | A | - |
| Example 170 | Calcium acetate | Type III AFP | 0.1 | 0.5 | 0.23 | - | A | - |
| Example 171 | Calcium acetate | Type III AFP | 0.5 | 0.5 | 0.40 | 0.35 | A | A |
| Example 172 | Sodium chloride | Type III AFP | 0.1 | 0.5 | 0.23 | - | A | - |
| Example 173 | Sodium chloride | Type III AFP | 0.5 | 0.5 | 0.29 | - | A | - |
| Example 174 | Sodium hydrogen carbonate | Type III AFP | 0.1 | 0.5 | 0.16 | - | A | - |
| Example 175 | Sodium hydrogen carbonate | Type III AFP | 0.5 | 0.5 | 0.34 | - | A | - |
| Example 176 | Sodium carbonate | Type III AFP | 0.1 | 0.5 | 0.23 | - | A | - |
| Example 177 | Sodium carbonate | Type III AFP | 0.5 | 0.5 | 0.31 | - | A | - |
| Example 178 | Sodium sulfite | Type III AFP | 0.1 | 0.5 | 0.24 | - | A | |
| Example 179 | Sodium sulfite | Type III AFP | 0.5 | 0.5 | 0.46 | 0.27 | A | A |
| Example 180 | Sodium thiosulfate | Type III AFP | 0.1 | 0.5 | 0.20 | - | A | - |
| Example 181 | Sodium thiosulfate | Type III AFP | 0.5 | 0.5 | 0.35 | - | A | - |
| Example 182 | Sodium nitrate | Type III AFP | 0.1 | 0.5 | 0.16 | - | A | - |
| Example 183 | Sodium nitrate | Type III AFP | 0.5 | 0.5 | 0.26 | - | A | - |

**[Table 14]**

| Example | Additive | AFP type (kind) | AFP concentration (mass%) | Additive concentration (M) | Thermal hysteresis activity (°C) | Thermal hysteresis activity h(°C) | Promotion effect | Heat resistance effect |
|---|---|---|---|---|---|---|---|---|
| Example 184 | Sodium dihydrogen phosphate | Type III AFP | 0.1 | 0.5 | 0.17 | - | A | - |
| Example 185 | Sodium dihydrogen phosphate | Type III AFP | 0.5 | 0.5 | 0.34 | - | A | - |
| Example 186 | Potassium chloride | Type III AFP | 0.1 | 0.5 | 0.17 | - | A | - |
| Example 187 | Potassium chloride | Type III AFP | 0.5 | 0.5 | 0.25 | - | A | - |
| Example 188 | Potassium acetate | Type III AFP | 0.1 | 0.5 | 0.18 | - | A | - |
| Example 189 | Potassium acetate | Type III AFP | 0.5 | 0.5 | 0.30 | - | A | - |
| Example 190 | Potassium carbonate | Type III AFP | 0.1 | 0.5 | 0.18 | - | A | - |
| Example 191 | Potassium carbonate | Type III AFP | 0.5 | 0.5 | 0.26 | - | A | - |
| Example 192 | Potassium nitrate | Type III AFP | 0.1 | 0.5 | 0.15 | - | A | - |
| Example 193 | Potassium nitrate | Type III AFP | 0.5 | 0.5 | 0.23 | - | A | - |
| Example 194 | Calcium chloride | Type III AFP | 0.1 | 0.5 | 0.23 | - | A | - |
| Example 195 | Calcium chloride | Type III AFP | 0.5 | 0.5 | 0.36 | - | A | - |
| Example 196 | Calcium nitrate | Type III AFP | 0.1 | 0.5 | 0.19 | - | A | - |
| Example 197 | Calcium nitrate | Type III AFP | 0.5 | 0.5 | 0.31 | - | A | - |
| Example 198 | Ammonium chloride | Type III AFP | 0.1 | 0.5 | 0.18 | - | A | - |
| Example 199 | Ammonium chloride | Type III AFP | 0.5 | 0.5 | 0.45 | - | A | - |
| Example 200 | Ammonium sulfate | Type III AFP | 0.1 | 0.5 | 0.35 | - | A | - |
| Example 201 | Ammonium sulfate | Type III AFP | 0.5 | 0.5 | 0.52 | - | A | - |
| Example 202 | Ammonium nitrate | Type III AFP | 0.1 | 0.5 | 0.28 | - | A | - |
| Example 203 | Ammonium nitrate | Type III AFP | 0.5 | 0.5 | 0.43 | - | A | - |
| Example 204 | Ammonium acetate | Type III AFP | 0.1 | 0.5 | 0.32 | - | A | - |
| Example 205 | Ammonium acetate | Type III AFP | 0.5 | 0.5 | 0.45 | 0.39 | A | A |

**[Table 15]**

| Example | Additive | AFP type (kind) | AFP concentration (mass%) | Additive concentration (M) | Thermal hysteresis activity (°C) | Thermal hysteresis activity h(°C) | Promotion effect | Heat resistance effect |
|---|---|---|---|---|---|---|---|---|
| Example 206 | Ammonium dihydrogen phosphate | Type III AFP | 0.1 | 0.5 | 0.31 | - | A | - |
| Example 207 | Ammonium dihydrogen phosphate | Type III AFP | 0.5 | 0.5 | 0.55 | - | A | - |
| Example 208 | Triammonium citrate | Type III AFP | 0.1 | 0.5 | 0.34 | - | A | - |
| Example 209 | Triammonium citrate | Type III AFP | 0.5 | 0.5 | 0.64 | - | A | - |
| Example 210 | Diammonium citrate | Type III AFP | 0.1 | 0.5 | 0.42 | - | A | - |
| Example 211 | Diammonium citrate | Type III AFP | 0.5 | 0.5 | 0.59 | - | A | - |
| Example 212 | Diammonium hydrogen phosphate | Type III AFP | 0.1 | 0.5 | 0.28 | - | A | - |
| Example 213 | Diammonium hydrogen phosphate | Type III AFP | 0.5 | 0.5 | 0.47 | - | A | - |
| Example 214 | Ammonium sulfite (monohydrate) | Type III AFP | 0.1 | 0.5 | 0.36 | - | A | - |
| Example 215 | Ammonium sulfite (monohydrate) | Type III AFP | 0.5 | 0.5 | 0.58 | - | A | - |
| Example 216 | Ammonium thiosulfate | Type III AFP | 0.1 | 0.5 | 0.33 | - | A | - |
| Example 217 | Ammonium thiosulfate | Type III AFP | 0.5 | 0.5 | 0.50 | - | A | - |

**[Table 16]**

| Example | Additive | AFP type (kind) | AFP concentration (mass%) | Additive concentration (M) | Thermal hysteresis activity (°C) | Thermal hysteresis activity h (°C) | Promotion effect | Heat resistance effect |
|---|---|---|---|---|---|---|---|---|
| Example 218 | Acetic acid | AFGP | 0.1 | 0.5 | 0.11 | - | B | - |
| Example 219 | Acetic acid | AFGP | 0.5 | 0.5 | 0.20 | - | A | - |
| Example 220 | L-ascorbic acid | AFGP | 0.1 | 0.5 | 0.16 | - | A | - |
| Example 221 | L-ascorbic acid | AFGP | 0.5 | 0.5 | 0.15 | 0.18 | B | B |
| Example 222 | Citric acid | AFGP | 0.1 | 0.5 | 0.12 | - | B | - |
| Example 223 | Citric acid | AFGP | 0.5 | 0.5 | 0.18 | - | A | - |
| Example 224 | Maleic acid | AFGP | 0.1 | 0.5 | 0.14 | - | A | - |
| Example 225 | Maleic acid | AFGP | 0.5 | 0.5 | 0.26 | - | A | - |
| Example 226 | Tartaric acid | AFGP | 0.1 | 0.5 | 0.10 | - | B | - |
| Example 227 | Tartaric acid | AFGP | 0.5 | 0.5 | 0.22 | - | A | - |
| Example 228 | Trisodium citrate | AFGP | 0.01 | 0.5 | 0.17 | 0.00 | A | c |
| Example 229 | Trisodium citrate | AFGP | 0.1 | 0.5 | 0.29 | - | A | - |
| Example 230 | Trisodium citrate | AFGP | 0.5 | 0.5 | 0.64 | - | A | - |
| Example 231 | Sodium acetate | AFGP | 0.01 | 0.5 | 0.13 | - | B | - |
| Example 232 | Sodium acetate | AFGP | 0.1 | 0.5 | 0.19 | - | A | - |
| Example 233 | Sodium acetate | AFGP | 0.5 | 0.5 | 0.25 | - | A | - |
| Example 234 | Sodium pyruvate | AFGP | 0.1 | 0.5 | 0.16 | - | A | - |
| Example 235 | Sodium pyruvate | AFGP | 0.5 | 0.5 | 0.26 | - | A | - |
| Example 236 | Calcium acetate | AFGP | 0.01 | 0.5 | 0.13 | - | B | - |
| Example 237 | Calcium acetate | AFGP | 0.1 | 0.5 | 0.20 | - | A | - |
| Example 238 | Calcium acetate | AFGP | 0.5 | 0.5 | 0.32 | 0.32 | A | A |
| Example 239 | Sodium chloride | AFGP | 0.01 | 0.5 | 0.14 | - | A | - |
| Example 240 | Sodium chloride | AFGP | 0.1 | 0.5 | 0.21 | - | A | - |
| Example 241 | Sodium chloride | AFGP | 0.5 | 0.5 | 0.24 | - | A | - |
| Example 242 | Sodium hydrogen carbonate | AFGP | 0.1 | 0.5 | 0.13 | - | B | - |
| Example 243 | Sodium hydrogen carbonate | AFGP | 0.5 | 0.5 | 0.22 | - | A | - |

**[Table 17]**

| Example | Additive | AFP type (kind) | AFP concentration (mass%) | Additive concentration (M) | Thermal hysteresis activity (°C) | Thermal hysteresis activity h (°C) | Promotion effect | Heat resistance effect |
|---|---|---|---|---|---|---|---|---|
| Example 244 | Sodium carbonate | AFGP | 0.1 | 0.5 | 0.18 | - | A | - |
| Example 245 | Sodium carbonate | AFGP | 0.5 | 0.5 | 0.36 | - | A | - |
| Example 246 | Sodium sulfite | AFGP | 0.01 | 0.5 | 0.15 | - | A | - |
| Example 247 | Sodium sulfite | AFGP | 0.1 | 0.5 | 0.20 | - | A | - |
| Example 248 | Sodium sulfite | AFGP | 0.5 | 0.5 | 0.50 | 0.32 | A | A |
| Example 249 | Sodium thiosulfate | AFGP | 0.01 | 0.5 | 0.14 | - | A | - |
| Example 250 | Sodium thiosulfate | AFGP | 0.1 | 0.5 | 0.22 | - | A | - |
| Example 251 | Sodium thiosulfate | AFGP | 0.5 | 0.5 | 0.38 | - | A | - |
| Example 252 | Sodium nitrate | AFGP | 0.1 | 0.5 | 0.18 | - | A | - |
| Example 253 | Sodium nitrate | AFGP | 0.5 | 0.5 | 0.25 | - | A | - |
| Example 254 | Sodium dihydrogen phosphate | AFGP | 0.1 | 0.5 | 0.13 | - | B | - |
| Example 255 | Sodium dihydrogen phosphate | AFGP | 0.5 | 0.5 | 0.28 | - | A | - |
| Example 256 | Potassium chloride | AFGP | 0.1 | 0.5 | 0.24 | - | A | - |
| Example 257 | Potassium chloride | AFGP | 0.5 | 0.5 | 0.21 | - | A | - |
| Example 258 | Potassium acetate | AFGP | 0.1 | 0.5 | 0.15 | - | A | - |
| Example 259 | Potassium acetate | AFGP | 0.5 | 0.5 | 0.43 | - | A | - |
| Example 260 | Potassium carbonate | AFGP | 0.1 | 0.5 | 0.19 | - | A | - |
| Example 261 | Potassium carbonate | AFGP | 0.5 | 0.5 | 0.38 | - | A | - |
| Example 262 | Potassium nitrate | AFGP | 0.1 | 0.5 | 0.17 | - | A | - |
| Example 263 | Potassium nitrate | AFGP | 0.5 | 0.5 | 0.20 | - | A | - |
| Example 264 | Calcium chloride | AFGP | 0.1 | 0.5 | 0.14 | - | A | - |
| Example 265 | Calcium chloride | AFGP | 0.5 | 0.5 | 0.21 | - | A | - |
| Example 266 | Calcium nitrate | AFGP | 0.1 | 0.5 | 0.17 | - | A | - |
| Example 267 | Calcium nitrate | AFGP | 0.5 | 0.5 | 0.25 | - | A | - |
| Example 268 | Ammonium chloride | AFGP | 0.1 | 0.5 | 0.18 | - | A | - |
| Example 269 | Ammonium chloride | AFGP | 0.5 | 0.5 | 0.36 | - | A | - |
| Example 270 | Ammonium chloride | AFGP | 0.5 | 1 | 0.40 | - | A | - |
| Example 271 | Ammonium chloride | AFGP | 0.5 | 2 | 0.42 | - | A | - |

**[Table 18]**

| Example | Additive | AFP type (kind) | AFP concentration (mass%) | Additive concentration (M) | Thermal hysteresis activity (°C) | Thermal hysteresis activity h (°C) | Promotion effect | Heat resistance effect |
|---|---|---|---|---|---|---|---|---|
| Example 272 | Ammonium sulfate | AFGP | 0.5 | 0.5 | 0.06 | - | C | - |
| Example 273 | Ammonium sulfate | AFGP | 1 | 0.5 | 0.59 | - | A | - |
| Example 274 | Ammonium sulfate | AFGP | 5 | 0.5 | 1.24 | - | A | - |
| Example 275 | Ammonium sulfate | AFGP | 0.5 | 1 | 1.14 | - | A | - |
| Example 276 | Ammonium sulfate | AFGP | 1 | 1 | 1.15 | - | A | - |
| Example 277 | Ammonium sulfate | AFGP | 5 | 1 | 2.02 | - | A | - |
| Example 278 | Ammonium sulfate | AFGP | 0.5 | 2 | 2.75 | - | A | - |
| Example 279 | Ammonium sulfate | AFGP | 1 | 2 | 2.77 | - | A | - |
| Example 280 | Ammonium sulfate | AFGP | 5 | 2 | 5.14 | - | A | - |
| Example 281 | Ammonium sulfate | AFGP | 0.5 | 3 | 5.91 | - | A | - |
| Example 282 | Ammonium sulfate | AFGP | 1 | 3 | 6.11 | - | A | - |
| Example 283 | Ammonium sulfate | AFGP | 5 | 3 | 7.51 | - | A | - |
| Example 284 | Ammonium nitrate | AFGP | 0.1 | 0.5 | 0.18 | - | A | - |
| Example 285 | Ammonium nitrate | AFGP | 0.5 | 0.5 | 0.39 | - | A | - |
| Example 286 | Ammonium hydrogen carbonate | AFGP | 0.5 | 0.5 | 0.27 | - | A | - |
| Example 287 | Ammonium hydrogen carbonate | AFGP | 0.5 | 1 | 0.36 | - | A | - |
| Example 288 | Ammonium acetate | AFGP | 0.1 | 0.5 | 0.34 | - | A | - |
| Example 289 | Ammonium acetate | AFGP | 0.5 | 0.5 | 0.74 | 0.55 | A | A |
| Example 290 | Ammonium acetate | AFGP | 0.5 | 1 | 0.54 | - | A | - |
| Example 291 | Ammonium acetate | AFGP | 0.5 | 2 | 0.77 | - | A | - |
| Example 292 | Ammonium dihydrogen phosphate | AFGP | 0.5 | 0.5 | 0.44 | - | A | - |
| Example 293 | Ammonium dihydrogen phosphate | AFGP | 0.5 | 1 | 0.6 | - | A | - |
| Example 294 | Ammonium dihydrogen phosphate | AFGP | 0.5 | 2 | 1.00 | - | A | - |

**[Table 19]**

| Example | Additive | AFP type (kind) | AFP concentration (mass%) | Additive concentration (M) | Thermal hysteresis activity (°C) | Thermal hysteresis activity h(°C) | Promotion effect | Heat resistance effect |
|---|---|---|---|---|---|---|---|---|
| Example 295 | Triammonium citrate | AFGP | 0.1 | 0.5 | 0.45 | - | A | - |
| Example 296 | Triammonium citrate | AFGP | 0.5 | 0.5 | 0.77 | - | A | - |
| Example 297 | Triammonium citrate | AFGP | 0.5 | 1 | 1.33 | - | A | - |
| Example 298 | Triammonium citrate | AFGP | 0.5 | 2 | 2.07 | - | A | - |
| Example 299 | Diammonium citrate | AFGP | 0.1 | 0.5 | 0.49 | - | A | - |
| Example 300 | Diammonium citrate | AFGP | 0.5 | 0.5 | 0.76 | - | A | - |
| Example 301 | Diammonium citrate | AFGP | 0.5 | 1 | 1.06 | - | A | - |
| Example 302 | Diammonium citrate | AFGP | 0.5 | 2 | 2.23 | - | A | - |
| Example 303 | Diammonium hydrogen phosphate | AFGP | 0.1 | 0.5 | 0.33 | - | A | - |
| Example 304 | Diammonium hydrogen phosphate | AFGP | 0.5 | 0.5 | 0.51 | - | A | - |
| Example 305 | Diammonium hydrogen phosphate | AFGP | 0.5 | 1 | 0.83 | - | A | - |
| Example 306 | Diammonium hydrogen phosphate | AFGP | 0.5 | 2 | 2.17 | - | A | - |
| Example 307 | Ammonium sulfite (monohydrate) | AFGP | 0.1 | 0.5 | 0.40 | - | A | - |
| Example 308 | Ammonium sulfite (monohydrate) | AFGP | 0.5 | 0.5 | 0.48 | - | A | - |
| Example 309 | Ammonium sulfite (monohydrate) | AFGP | 0.5 | 1 | 0.88 | - | A | - |
| Example 310 | Ammonium sulfite (monohydrate) | AFGP | 0.5 | 2 | 1.56 | - | A | - |
| Example 311 | Ammonium thiosulfate | AFGP | 0.1 | 0.5 | 0.37 | - | A | - |
| Example 312 | Ammonium thiosulfate | AFGP | 0.5 | 0.5 | 0.46 | - | A | - |
| Example 313 | Ammonium thiosulfate | AFGP | 0.5 | 1 | 0.78 | - | A | - |
| Example 314 | Ammonium thiosulfate | AFGP | 0.5 | 2 | 2.00 | - | A | - |

In Examples 1 to 314 shown in the above Tables 3 to 19, the promotion effect in the case where the thermal hysteresis activity was promoted to 1.5 times or more compared to Comparative Examples was rated as "A"; the promotion effect in the case where the thermal hysteresis activity was promoted by more than 1.0 times and less than 1.5 times was rated as "B"; and the promotion effect in the case where the thermal hysteresis activity was 1.0 times or less and was not promoted was rated as "C".
Furthermore, in Examples 1 to 92 shown in the Tables 3 to 9, the heat resistance effect in the case where the thermal hysteresis activity h was 1.5 times or more compared to Comparative Examples was rated as "A"; the heat resistance effect in the case where the thermal hysteresis activity h was more than 1.0 times and less than 1.5 times was rated as "B"; and the promotion effect in the case where the thermal hysteresis activity h was 1.0 times or less and was not promoted was rated as "C". When the promotion effect or heat resistance effect was not evaluated, the example was indicated as "-".

From the results described above, it is clear that the method of increasing thermal hysteresis activity, the method of reducing thermal deactivation of the thermal hysteresis activity, and the composition for increasing thermal hysteresis activity according to the invention are excellent in the increasing effect and/or the reducing effect.

### INDUSTRIAL APPLICABILITY

The method of increasing thermal hysteresis activity, the method of reducing thermal deactivation of the thermal hysteresis activity, and the composition for increasing thermal hysteresis activity of the invention can be widely used, when applied to foods or industrial chemical liquids containing water, for suppressing the growth of ice crystals or recrystallization of ice when the foods or the industrial chemical liquids are stored cold, smoothening the melt-in-the-mouth of the foods, or preventing any damage caused by the growth of ice in the foods or the industrial chemical liquids.

## Claims

1. A method of increasing a thermal hysteresis activity exhibited by a composition comprising an antifreeze protein and water, the method comprising:
incorporating the antifreeze protein and any one or more substances selected from a following additive group A into the water:
additive group A: an organic acid, an organic acid salt, an inorganic salt, an amino acid, an amino acid salt, an ammonium salt, a sugar, a sugar alcohol, urea, guanidine hydrochloride, spermidine, spermine, and N,N-dimethylformamide.

2. A method of reducing a post-heat treatment deactivation of a thermal hysteresis activity exhibited by a composition comprising an antifreeze protein and water, the method comprising:
incorporating the antifreeze protein and any one or more substances selected from the following additive group B into the water:
additive group B: an organic acid, an organic acid salt, an inorganic salt, an amino acid, an amino acid salt, an ammonium salt, a sugar, a sugar alcohol, urea, guanidine hydrochloride, acetone, dioxane, 2-chloroethanol, and N,N-dimethylformamide.

3. A composition for increasing thermal hysteresis activity, the composition comprising: an antifreeze protein;
water; and
an additive,
wherein the additive comprises any one or more substances selected from the group consisting of an organic acid, an organic acid salt, an inorganic salt, an amino acid, an amino acid salt, an ammonium salt, a sugar, a sugar alcohol, urea, guanidine hydrochloride, spermidine, spermine, acetone, dioxane, 2-chloroethanol, and N,N-dimethylformamide.

4. The method of increasing the thermal hysteresis activity according to Claim 1, wherein the antifreeze protein is a fish-derived protein.

5. The method of reducing the post-heat treatment deactivation of the thermal hysteresis activity according to Claim 2, wherein the antifreeze protein is a fish-derived protein.

6. The composition for increasing thermal hysteresis activity according to Claim 3, wherein the antifreeze protein is a fish-derived protein.

7. The method of increasing the thermal hysteresis activity according to Claim 4, wherein the antifreeze protein is a Type I antifreeze protein.

8. The method of increasing the thermal hysteresis activity according to Claim 4, wherein the antifreeze protein is a Type II antifreeze protein.

9. The method of increasing the thermal hysteresis activity according to Claim 4, wherein the antifreeze protein is a Type III antifreeze protein.

10. The method of increasing the thermal hysteresis activity according to Claim 4, wherein the antifreeze protein is an AFGP type antifreeze protein.

11. The method of reducing the post-heat treatment deactivation of the thermal hysteresis activity according to Claim 5, wherein the antifreeze protein is a Type I antifreeze protein.

12. The method of reducing the post-heat treatment deactivation of the thermal hysteresis activity according to Claim 5, wherein the antifreeze protein is a Type II antifreeze protein.

13. The method of reducing the post-heat treatment deactivation of the thermal hysteresis activity according to Claim 5, wherein the antifreeze protein is a Type III antifreeze protein.

14. The method of reducing the post-heat treatment deactivation of the thermal hysteresis activity according to Claim 5, wherein the antifreeze protein is an AFGP type antifreeze protein.

15. The method of increasing the thermal hysteresis activity according to Claim 1, wherein a concentration of the antifreeze protein comprised in a mixture of water, the antifreeze protein and the additive is 0.01 mass% to 10 mass%.

16. The method of reducing the post-heat treatment deactivation of the thermal hysteresis activity according to Claim 2, wherein a concentration of the antifreeze protein comprised in a mixture of water, the antifreeze protein and the additive is 0.01 mass% to 10 mass%.

17. The composition for increasing thermal hysteresis activity according to Claim 3, wherein a concentration of the antifreeze protein comprised in the composition for increasing thermal hysteresis activity is 0.01 mass% to 10 mass%.
